# EUROPEAN PATENT SPECIFICATION

(11) **EP 3 359 689 B1**
(45) Date of publication and mention of the grant of the patent: **14.04.2021**
(21) Application number: 16852952.7
(22) Date of filing: 07.10.2016
(51) Int. Cl.: C12Q 1/6886, C40B 30/04, C40B 40/06, G01N 33/48

(54) **USE OF A GENETIC SIGNATURE DIAGNOSTICALLY TO EVALUATE TREATMENT STRATEGIES FOR PROSTATE CANCER**
DIAGNOSTISCHE VERWENDUNG EINES EINER GENETISCHEN SIGNATUR ZUR BEWERTUNG VON BEHANDLUNGSSTRATEGIEN FÜR PROSTATAKREBS
UTILISATION DE MANIÈRE DIAGNOSTIQUE D'UNE SIGNATURE GÉNÉTIQUE POUR ÉVALUER LES STRATÉGIES DE TRAITEMENT DU CANCER DE LA PROSTATE

(30) Priority: 08.10.2015 US 201562239181 P
(43) Date of publication of application: 15.08.2018
(73) Proprietor: Decipher Biosciences, Inc., San Diego, CA 92121 (US)
(72) Inventor: AL-DEEN ASHAB, Hussam, Vancouver, British Columbia V6B 2W9 (CA); ERHO, Nicholas, Vancouver, British Columbia V6B 6H4 (CA); ALSHALALFA, Mohammed, New Westminster, British Columbia V3L 0A4 (CA); DAVICIONI, Elai, La Jolla, California 92037 (US)
(74) Representative: Cornish, Kristina Victoria Joy
(86) International application number: PCT/CA2016/051180
(87) International publication number: WO 2017/059549

(56) References cited:
- WO-A1-2013/116742
- WO-A2-2014/028884
- ANDREA LUNARDI ET AL: "A co-clinical approach identifies mechanisms and potential therapies for androgen deprivation resistance in prostate cancer", NATURE GENETICS., vol. 45, no. 7, 1 July 2013 (2013-07-01), pages 747-755, XP055579276, NEW YORK, US ISSN: 1061-4036, DOI: 10.1038/ng.2650
- AFFYMETRIX: "GeneChip exon array system for human, mouse, and rat", INTERNET CITATION, 1 January 2006 (2006-01-01), XP002480421, Retrieved from the Internet: URL:http://www.affymetrix.com/products/arr ays/exon_application.affx
- MICHAEL STANBROUGH ET AL: "Increased expression of genes converting adrenal androgens to testosterone in androgen-independent prostate cancer", CANCER RESEARCH, PROCEEDINGS: AACR ANNUAL MEETING 2014; APRIL 5-9, 2014; SAN DIEGO, CA, vol. 66, no. 5, 1 March 2006 (2006-03-01), pages 2815-2825, XP002639099, ISSN: 0008-5472, DOI: 10.1158/0008-5472.CAN-05-4000
- KEN-ICHI TAKAYAMA ET AL: "TACC2 Is an Androgen-Responsive Cell Cycle Regulator Promoting Androgen-Mediated and Castration-Resistant Growth of Prostate Cancer", MOLECULAR ENDOCRINOLOGY, vol. 26, no. 5, 1 May 2012 (2012-05-01), pages 748-761, XP055579856, US ISSN: 0888-8809, DOI: 10.1210/me.2011-1242
- A DAHLMAN ET AL: "Effect of androgen deprivation therapy on the expression of prostate cancer biomarkers MSMB and MSMB-binding protein CRISP3", PROSTATE CANCER AND PROSTATIC DISEASE, vol. 13, no. 4, 3 August 2010 (2010-08-03) , pages 369-375, XP055579877, BASINGSTOKE, GB ISSN: 1365-7852, DOI: 10.1038/pcan.2010.25
- ERHO ET AL.: 'Discovery and Validation of a Prostate Cancer Genomic Classifier that Predicts Early Metastasis Following Radical Prostatectomy' PLOS ONE vol. 8, no. 6, 24 June 2013, ISSN 1932-6203 page E66855., XP055371391
- DALELA ET AL.: 'Contemporary Role of the Decipher Test in Prostate Cancer Mangement: Current Practice and Future Perspectives' REVIEWS IN UROLOGY vol. 18, no. 1, 2016, ISSN 1523-6161 pages 1 - 9, XP055371396
- BAETKE ET AL.: 'Molecular Pathways Involved in Prostate Carcinogenesis: Insights from Public Microarray Datasets' PLOS ONE vol. 7, no. 11, 20 November 2012, ISSN 1932-6203 page E49831, XP055371397

## Description

### FIELD OF THE INVENTION

The present invention pertains to the field of personalized medicine and methods for diagnosing and treating prostate cancer. In particular, the invention relates to the use of a genetic signature to identify subjects in need of treatment for prostate cancer who will be resistant to androgen deprivation therapy (ADT).

### BACKGROUND OF THE INVENTION

Cancer is the uncontrolled growth of abnormal cells anywhere in a body. The abnormal cells are termed cancer cells, malignant cells, or tumor cells. Many cancers and the abnormal cells that compose the cancer tissue are further identified by the name of the tissue that the abnormal cells originated from (for example, prostate cancer). Cancer cells can proliferate uncontrollably and form a mass of cancer cells. Cancer cells can break away from this original mass of cells, travel through the blood and lymph systems, and lodge in other organs where they can again repeat the uncontrolled growth cycle. This process of cancer cells leaving an area and growing in another body area is often termed metastatic spread or metastatic disease. For example, if prostate cancer cells spread to a bone (or anywhere else), it can mean that the individual has metastatic prostate cancer.

Standard clinical parameters such as tumor size, grade, lymph node involvement and tumor-node-metastasis (TNM) staging (American Joint Committee on Cancer) may correlate with outcome and serve to stratify subjects with respect to (neo)adjuvant chemotherapy, immunotherapy, antibody therapy and/or radiotherapy regimens. Incorporation of molecular markers in clinical practice may define tumor subtypes that are more likely to respond to targeted therapy. However, stage-matched tumors grouped by histological or molecular subtypes may respond differently to the same treatment regimen. Additional key genetic and epigenetic alterations may exist with important etiological contributions. A more detailed understanding of the molecular mechanisms and regulatory pathways at work in cancer cells and the tumor microenvironment (TME) could dramatically improve the design of novel anti-tumor drugs and inform the selection of optimal therapeutic strategies. The development and implementation of diagnostic, prognostic and therapeutic biomarkers to characterize the biology of each tumor may assist clinicians in making important decisions with regard to individual subject care and treatment.

This background information is provided for the purpose of making known information believed by the applicant to be of possible relevance to the present invention. No admission is necessarily intended, nor should be construed, that any of the preceding information constitutes prior art against the present invention.

### SUMMARY OF THE INVENTION

The invention is as set out in the appended claims. The present invention is based on the discovery of a genetic signature that is useful for identifying subjects in need of treatment for prostate cancer who are resistant to androgen deprivation therapy (ADT).

The present invention provides a diagnostic method for predicting resistance to ADT for a subject who has prostate cancer, the method comprising measuring the level of expression of a plurality of biomarker genes comprising SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12, NLRP13, MPP7, CFL1, DESI2, OR51E2, KCNMB1, DLGAP1, SPRR1A, CROT, KIFC1, POLD4, CASP2, WHSC1, MPZL2, NAV1, RNF168, FOXM1, ZC3H11A, FAM3D, KCNK17, PLXNA2, SUOX, ANP32E, REST, NKX2.2, RBBP8, NSMCE4A, H19, ATP1A2, PLXNC1, NUP62, ACAA2, ADH1C, THYN1, COX7A2L and MAP1B in a biological sample obtained from a subject; and calculating an ADT resistance signature (ARS) score based on the level of expression of the plurality of biomarkers, wherein a higher ARS score for the subject compared to reference value ranges for a control subject indicates that the subject is resistant to ADT.

Described herein, the plurality of biomarker genes is selected from SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12 and NLRP13.

Described herein is a diagnostic method for predicting resistance to ADT for a subject who has prostate cancer, the method comprising measuring the level of expression of a plurality of biomarker genes comprising DAND5, GABRB3, KLK12, NAV2, POGK, RIMS2, SNCAIP, TET1, TMEM176A, ELL2, ADAMTS14, CDKN2C, KCNMB2, MUC1, PLEKHH2, TNIK, AGTR1, BAG3, C10orf81, C8orf4, CRISP2, GALM, GHR, POLD4, RRAS, SPATA13, TMEFF2, ARHGAP11A, ASB16, CASP2, FAM57B, FOXM1, GNAZ, AC084018.1, NAV1, RRM2, TFAP4, HEPN1, KIF1C, MPDU1, RLN1, SELE, WDR93, ATF5, HEY2, NUP210, CDH3, CREB3L1, SLC35F2, TRPV6, FAM134A, NSMCE4A or a combination thereof in a biological sample obtained from a subject; and calculating an ADT resistance signature (ARS) score based on the level of expression of the plurality of biomarkers, wherein a higher ARS score for the subject compared to reference value ranges for a control subject indicates that the subject is resistant to ADT.

Described herein, the plurality of biomarker genes are selected from DAND5, GABRB3, RIMS2, SNCAIP, TMEM176A, KCNMB2, PLEKHH2, AGTR1, BAG3, C10orf81, C8orf4, CRISP2, GALM, GHR, RRAS, SPATA13, TMEFF2, ARHGAP11A, GNAZ, AC084018.1, RRM2, TFAP4, HEPN1, MPDU1, RLN1, SELE, WDR93, ATF5, HEY2, CREB3L1, SLC35F2, FAM134A, NSMCE4A or a combination thereof. Described herein, the plurality of genes further comprises one or more genes selected from KLK12, NAV2, POGK, TET1, ELL2, ADAMTS14, CDKN2C, MUC1, TNIK, POLD4, ASB16, CASP2, FAM57B, FOXM1, NAV1, KIF1C, NUP210, CDH3, and TRPV6.

Described herein, the plurality of genes comprises DAND5, GABRB3, KLK12, NAV2, POGK, RIMS2, SNCAIP, TET1, TMEM176A, ELL2, ADAMTS14, CDKN2C, KCNMB2, MUC1, PLEKHH2, TNIK, AGTR1, BAG3, C10orf81, C8orf4, CRISP2, GALM, GHR, POLD4, RRAS, SPATA13, TMEFF2, ARHGAP11A, ASB16, CASP2, FAM57B, FOXM1, GNAZ, AC084018.1, NAV1, RRM2, TFAP4, HEPN1, KIF1C, MPDU1, RLN1, SELE, WDR93, ATF5, HEY2, NUP210, CDH3, CREB3L1, SLC35F2, TRPV6, FAM134A, NSMCE4A or a combination thereof.

In yet other embodiments, the diagnostic method is performed while the subject is undergoing ADT or after treatment of the subject with ADT. In other embodiments, the method is performed after the subject undergoes radical prostatectomy.

In certain embodiments, the prostate cancer in the subject is adenocarcinoma, small cell prostate cancer, non-small cell prostate cancer, neuroendocrine prostate cancer, or metastatic castration resistant prostate cancer.

In another embodiment, the method further comprises predicting survival of a subject undergoing ADT for treatment of prostate cancer, wherein a higher ARS score for the subject compared to reference value ranges for a control subject indicates that the subject will have a shorter period of metastasis-free survival compared to a subject who is not resistant to ADT.

In some embodiments, the biological sample obtained from the subject is a prostate biopsy or tumor sample. In other embodiments, the biological sample is obtained from bodily fluid or tissue of the subject that contains cancer cells. In certain embodiments, nucleic acids comprising biomarker gene sequences are further isolated from the biological sample, and/or purified, and/or amplified prior to analysis.

In some embodiments, the expression level of biomarker nucleic acids is determined by microarray analysis, polymerase chain reaction (PCR), reverse transcriptase polymerase chain reaction (RT-PCR), a Northern blot, or serial analysis of gene expression (SAGE).

Also described is ADT for use in a method of treating prostate cancer, the method comprising determining whether or not the subject is resistant to ADT, as described herein; and administering ADT to the subject if the subject is not identified as resistant to ADT, or administering a cancer treatment other than ADT to the subject if the subject is identified as resistant to ADT. If the subject is identified as resistant to ADT, the subject may be administered a cancer treatment comprising, for example, surgery, radiation therapy, chemotherapy, immunotherapy, or biologic therapy, or any combination thereof.

Described herein is a kit for measuring expression levels of biomarker genes for identifying subjects resistant to ADT, as described herein. The kit may include one or more agents for measuring expression levels of biomarker genes (e.g., hybridization probes, PCR primers, or microarray), a container for holding a biological sample comprising prostate cancer cells isolated from a human subject for testing, and printed instructions for reacting the agents with the biological sample or a portion of the biological sample to determine whether or not the subject is resistant to ADT. The agents may be packaged in separate containers. The kit may further comprise one or more control reference samples or other reagents for measuring gene expression (e.g., reagents for performing PCR, RT-PCR, microarray analysis, a Northern blot, or SAGE).

Described herein, the kit comprises agents for measuring levels of expression of biomarker genes comprising SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12, NLRP13, MPP7, CFL1, DESI2, OR51E2, KCNMB1, DLGAP1, SPRR1A, CROT, KIFC1, POLD4, CASP2, WHSC1, MPZL2, NAV1, RNF168, FOXM1, ZC3H11A, FAM3D, KCNK17, PLXNA2, SUOX, ANP32E, REST, NKX2.2, RBBP8, NSMCE4A, H19, ATP1A2, PLXNC1, NUP62, ACAA2, ADH1C, THYN1, COX7A2L, MAP1B or a combination thereof. Described herein, the kit comprises agents for measuring levels of expression of biomarker genes selected from SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12 and NLRP13.

Described herein, the kit comprises agents for measuring levels of expression of biomarker genes comprising DAND5, GABRB3, RIMS2, SNCAIP, TMEM176A, KCNMB2, PLEKHH2, AGTR1, BAG3, C10orf81, C8orf4, CRISP2, GALM, GHR, RRAS, SPATA13, TMEFF2, ARHGAP11A, GNAZ, AC084018.1, RRM2, TFAP4, HEPN1, MPDU1, RLN1, SELE, WDR93, ATF5, HEY2, CREB3L1, SLC35F2, FAM134A, NSMCE4A or a combination thereof.

Described herein, the kit comprises agents for measuring the levels of expression of one or more biomarker genes selected from KLK12, NAV2, POGK, TET1, ELL2, ADAMTS14, CDKN2C, MUC1, TNIK, POLD4, ASB16, CASP2, FAM57B, FOXM1, NAV1, KIF1C, NUP210, CDH3, and TRPV6.

Described herein, the kit comprises agents for measuring the levels of expression of the biomarker genes DAND5, GABRB3, KLK12, NAV2, POGK, RIMS2, SNCAIP, TET1, TMEM176A, ELL2, ADAMTS14, CDKN2C, KCNMB2, MUC1, PLEKHH2, TNIK, AGTR1, BAG3, C10orf81, C8orf4, CRISP2, GALM, GHR, POLD4, RRAS, SPATA13, TMEFF2, ARHGAP11A, ASB16, CASP2, FAM57B, FOXM1, GNAZ, AC084018.1, NAV1, RRM2, TFAP4, HEPN1, KIF1C, MPDU1, RLN1, SELE, WDR93, ATF5, HEY2, NUP210, CDH3, CREB3L1, SLC35F2, TRPV6, FAM134A, NSMCE4A or a combination thereof.

The significance of the expression levels of one or more biomarker genes may be evaluated using, for example, a T-test, P-value, KS (Kolmogorov Smirnov) P-value, accuracy, accuracy P-value, positive predictive value (PPV), negative predictive value (NPV), sensitivity, specificity, AUC, AUC P-value (Auc.pvalue), Wilcoxon Test P-value, Median Fold Difference (MFD), Kaplan Meier (KM) curves, survival AUC (survAUC), Kaplan Meier P-value (KM P-value), Univariable Analysis Odds Ratio P-value (uvaORPval), multivariable analysis Odds Ratio P-value (mvaORPval), Univariable Analysis Hazard Ratio P-value (uvaHRPval) and Multivariable Analysis Hazard Ratio P-value (mvaHRPval). The significance of the expression level of the one or more targets may be based on two or more metrics selected from the group comprising AUC, AUC P-value (Auc.pvalue), Wilcoxon Test P-value, Median Fold Difference (MFD), Kaplan Meier (KM) curves, survival AUC (survAUC), Univariable Analysis Odds Ratio P-value (uvaORPval), multivariable analysis Odds Ratio P-value (mvaORPval), Kaplan Meier P-value (KM P-value), Univariable Analysis Hazard Ratio P-value (uvaHRPval) or Multivariable Analysis Hazard Ratio P-value (mvaHRPval).

Described herein is a computer implemented method for predicting resistance to androgen deprivation therapy (ADT) for a subject who has prostate cancer, the computer performing steps comprising receiving inputted subject data comprising values for the levels of expression of biomarker genes comprising SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12, NLRP13, MPP7, CFL1, DESI2, OR51E2, KCNMB1, DLGAP1, SPRR1A, CROT, KIFC1, POLD4, CASP2, WHSC1, MPZL2, NAV1, RNF168, FOXM1, ZC3H11A, FAM3D, KCNK17, PLXNA2, SUOX, ANP32E, REST, NKX2.2, RBBP8, NSMCE4A, H19, ATP1A2, PLXNC1, NUP62, ACAA2, ADH1C, THYN1, COX7A2L, MAP1B or a combination thereof in a biological sample comprising cancer cells from the subject; analyzing the level of expression of each biomarker gene and comparing with respective reference value ranges for each biomarker gene; calculating an ARS score for the subject based on the levels of expression of the biomarker genes, wherein a higher ARS score for the subject compared to reference value ranges for a control subject indicates that the subject is resistant to ADT; and displaying information regarding whether or not the subject is resistant to ADT. Described herein, the inputted subject data further comprises values for the levels of expression of one or more biomarker genes selected from SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12 and NLRP13. Analyzing the level of expression of biomarker gene may comprise the use of a probe set. Analyzing the level of expression of biomarker gene may comprise the use of a classifier. The classifier may comprise a probe selection region (PSR). The classifier may comprise the use of an algorithm. The algorithm may comprise a machine learning algorithm. Assaying the expression level may also comprise sequencing the plurality of biomarker genes.

Described herein is a computer implemented method for predicting resistance to androgen deprivation therapy (ADT) for a subject who has prostate cancer, the computer performing steps comprising receiving inputted subject data comprising values for the levels of expression of biomarker genes comprising DAND5, GABRB3, RIMS2, SNCAIP, TMEM176A, KCNMB2, PLEKHH2, AGTR1, BAG3, C10orf81, C8orf4, CRISP2, GALM, GHR, RRAS, SPATA13, TMEFF2, ARHGAP11A, GNAZ, AC084018.1, RRM2, TFAP4, HEPN1, MPDU1, RLN1, SELE, WDR93, ATF5, HEY2, CREB3L1, SLC35F2, FAM134A, NSMCE4A or a combination thereof in a biological sample comprising cancer cells from the subject; analyzing the level of expression of each biomarker gene and comparing with respective reference value ranges for each biomarker gene; calculating an ARS score for the subject based on the levels of expression of the biomarker genes, wherein a higher ARS score for the subject compared to reference value ranges for a control subject indicates that the subject is resistant to ADT; and displaying information regarding whether or not the subject is resistant to ADT. Described herein, the inputted subject data further comprises values for the levels of expression of one or more biomarker genes selected from KLK12, NAV2, POGK, TET1, ELL2, ADAMTS14, CDKN2C, MUC1, TNIK, POLD4, ASB16, CASP2, FAM57B, FOXM1, NAV1, KIF1C, NUP210, CDH3, and TRPV6. Described herein, the inputted subject data comprises values for the levels of expression of biomarker genes comprising DAND5, GABRB3, KLK12, NAV2, POGK, RIMS2, SNCAIP, TET1, TMEM176A, ELL2, ADAMTS14, CDKN2C, KCNMB2, MUC1, PLEKHH2, TNIK, AGTR1, BAG3, C10orf81, C8orf4, CRISP2, GALM, GHR, POLD4, RRAS, SPATA13, TMEFF2, ARHGAP11A, ASB16, CASP2, FAM57B, FOXM1, GNAZ, AC084018.1, NAV1, RRM2, TFAP4, HEPN1, KIF1C, MPDU1, RLN1, SELE, WDR93, ATF5, HEY2, NUP210, CDH3, CREB3L1, SLC35F2, TRPV6, FAM134A, NSMCE4A or a combination thereof. Analyzing the level of expression of biomarker gene may comprise the use of a probe set. Analyzing the level of expression of biomarker gene may comprise the use of a classifier. The classifier may comprise a probe selection region (PSR). The classifier may comprise the use of an algorithm. The algorithm may comprise a machine learning algorithm. Assaying the expression level may also comprise sequencing the plurality of biomarker genes.

Described herein is a diagnostic system for predicting whether or not a subject who has prostate cancer is resistant to ADT, the diagnostic system comprising a storage component (memory) for storing data, wherein the storage component has instructions for calculating an ARS score for the subject stored therein; a computer processor for processing data, wherein the computer processor is coupled to the storage component and configured to execute the instructions stored in the storage component in order to receive subject data and analyze subject data according to one or more algorithms; and a display component for displaying information regarding the diagnosis of the subject.

Also described is a method comprising measuring the level of expression of a plurality of biomarker genes comprising SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12, NLRP13, MPP7, CFL1, DESI2, OR51E2, KCNMB1, DLGAP1, SPRR1A, CROT, KIFC1, POLD4, CASP2, WHSC1, MPZL2, NAV1, RNF168, FOXM1, ZC3H11A, FAM3D, KCNK17, PLXNA2, SUOX, ANP32E, REST, NKX2.2, RBBP8, NSMCE4A, H19, ATP1A2, PLXNC1, NUP62, ACAA2, ADH1C, THYN1, COX7A2L and MAP1B in a biological sample from a subject; calculating an ADT resistance signature (ARS) score based on the level of expression of the plurality of biomarkers; and administering a treatment to the subject. Described herein, the plurality of biomarker genes is selected from SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12 and NLRP13. In an additional aspect, the subject is undergoing ADT. In a further aspect, the method is performed after treatment of the subject with ADT. In certain aspects, the prostate cancer is adenocarcinoma, small cell prostate cancer, neuroendocrine prostate cancer or metastatic castration resistant prostate cancer. In one aspect, the method is performed after the subject undergoes radical prostatectomy. In another aspect, the biological sample is a biopsy or a tumor sample. In an additional aspect, measuring the level of expression comprises performing microarray analysis, polymerase chain reaction (PCR), reverse transcriptase polymerase chain reaction (RT-PCR), a Northern blot, or serial analysis of gene expression (SAGE). In some aspects the treatment is a cancer treatment comprising surgery, radiation therapy, chemotherapy, immunotherapy, biologic therapy, or any combination thereof.

Also described is ADT for use in a method of treating prostate cancer, the method comprising: measuring the level of expression of a plurality of biomarker genes comprising SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12, NLRP13, MPP7, CFL1, DESI2, OR51E2, KCNMB1, DLGAP1, SPRR1A, CROT, KIFC1, POLD4, CASP2, WHSC1, MPZL2, NAV1, RNF168, FOXM1, ZC3H11A, FAM3D, KCNK17, PLXNA2, SUOX, ANP32E, REST, NKX2.2, RBBP8, NSMCE4A, H19, ATP1A2, PLXNC1, NUP62, ACAA2, ADH1C, THYN1, COX7A2L and MAP1B in a biological sample from the subject; calculating an ADT resistance signature (ARS) score based on the level of expression of the plurality of biomarkers, wherein a higher ARS score for the subject compared to reference value ranges for a control subject indicates that the subject is resistant to ADT; and administering ADT to the subject if the subject is not identified as resistant to ADT, or administering a cancer treatment other than ADT to the subject if the subject is identified as resistant to ADT. Described herein, the plurality of biomarker genes is selected from SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12 and NLRP13. In one aspect, the subject identified as resistant to ADT is administered a cancer treatment comprising surgery, radiation therapy, chemotherapy, immunotherapy, biologic therapy, or any combination thereof.

Described herein, is a method comprising measuring the level of expression of a plurality of biomarker genes comprising DAND5, GABRB3, KLK12, NAV2, POGK, RIMS2, SNCAIP, TET1, TMEM176A, ELL2, ADAMTS14, CDKN2C, KCNMB2, MUC1, PLEKHH2, TNIK, AGTR1, BAG3, C10orf81, C8orf4, CRISP2, GALM, GHR, POLD4, RRAS, SPATA13, TMEFF2, ARHGAP11A, ASB16, CASP2, FAM57B, FOXM1, GNAZ, AC084018.1, NAV1, RRM2, TFAP4, HEPN1, KIF1C, MPDU1, RLN1, SELE, WDR93, ATF5, HEY2, NUP210, CDH3, CREB3L1, SLC35F2, TRPV6, FAM134A, NSMCE4A or a combination thereof in a biological sample obtained from a subject; calculating an ADT resistance signature (ARS) score based on the level of expression of the plurality of biomarkers; and administering a treatment to the subject. Described herein, the plurality of biomarker genes is selected from DAND5, GABRB3, RIMS2, SNCAIP, TMEM176A, KCNMB2, PLEKHH2, AGTR1, BAG3, C10orf81, C8orf4, CRISP2, GALM, GHR, RRAS, SPATA13, TMEFF2, ARHGAP11A, GNAZ, AC084018.1, RRM2, TFAP4, HEPN1, MPDU1, RLN1, SELE, WDR93, ATF5, HEY2, CREB3L1, SLC35F2, FAM134A, NSMCE4A or a combination thereof. Described herein, plurality of biomarker genes further comprises one or more genes selected from KLK12, NAV2, POGK, TET1, ELL2, ADAMTS14, CDKN2C, MUC1, TNIK, POLD4, ASB16, CASP2, FAM57B, FOXM1, NAV1, KIF1C, NUP210, CDH3, and TRPV6. Described herein, the plurality of biomarker genes comprises DAND5, GABRB3, KLK12, NAV2, POGK, RIMS2, SNCAIP, TET1, TMEM176A, ELL2, ADAMTS14, CDKN2C, KCNMB2, MUC1, PLEKHH2, TNIK, AGTR1, BAG3, C10orf81, C8orf4, CRISP2, GALM, GHR, POLD4, RRAS, SPATA13, TMEFF2, ARHGAP11A, ASB16, CASP2, FAM57B, FOXM1, GNAZ, AC084018.1, NAV1, RRM2, TFAP4, HEPN1, KIF1C, MPDU1, RLN1, SELE, WDR93, ATF5, HEY2, NUP210, CDH3, CREB3L1, SLC35F2, TRPV6, FAM134A, NSMCE4A or a combination thereof. In an additional aspect, the subject is undergoing ADT. In a further aspect, the method is performed after treatment of the subject with ADT. In certain aspects, the prostate cancer is adenocarcinoma, small cell prostate cancer, neuroendocrine prostate cancer or metastatic castration resistant prostate cancer. In one aspect, the method is performed after the subject undergoes radical prostatectomy. In another aspect, the biological sample is a biopsy or a tumor sample. In an additional aspect, measuring the level of expression comprises performing microarray analysis, polymerase chain reaction (PCR), reverse transcriptase polymerase chain reaction (RT-PCR), a Northern blot, or serial analysis of gene expression (SAGE). In some aspects the treatment is a cancer treatment comprising surgery, radiation therapy, chemotherapy, immunotherapy, biologic therapy, or any combination thereof.

Described herein is a method for treating a subject for prostate cancer, the method comprising: measuring the level of expression of a plurality of biomarker genes comprising DAND5, GABRB3, KLK12, NAV2, POGK, RIMS2, SNCAIP, TET1, TMEM176A, ELL2, ADAMTS14, CDKN2C, KCNMB2, MUC1, PLEKHH2, TNIK, AGTR1, BAG3, C10orf81, C8orf4, CRISP2, GALM, GHR, POLD4, RRAS, SPATA13, TMEFF2, ARHGAP11A, ASB16, CASP2, FAM57B, FOXM1, GNAZ, AC084018.1, NAV1, RRM2, TFAP4, HEPN1, KIF1C, MPDU1, RLN1, SELE, WDR93, ATF5, HEY2, NUP210, CDH3, CREB3L1, SLC35F2, TRPV6, FAM134A, NSMCE4A or a combination thereof in a biological sample obtained from the subject; calculating an ADT resistance signature (ARS) score based on the level of expression of the plurality of biomarkers, wherein a higher ARS score for the subject compared to reference value ranges for a control subject indicates that the subject is resistant to ADT; and administering ADT to the subject if the subject is not identified as resistant to ADT, or administering a cancer treatment other than ADT to the subject if the subject is identified as resistant to ADT. In one aspect, the subject identified as resistant to ADT is administered a cancer treatment comprising surgery, radiation therapy, chemotherapy, immunotherapy, biologic therapy, or any combination thereof.

These and other embodiments of the subject invention will readily occur to those of skill in the art in view of the disclosure herein.

### BRIEF DESCRIPTION OF THE DRAWINGS

FIG. 1 shows a study diagram showing the number of subjects used for training and validation in treated and untreated arms and their metastatic outcome.
FIG. 2 shows a flowchart showing steps performed to develop the ARS model.
FIG. 3 shows a model assessment in the training cohort showing subjects who failed ADT are at higher risk of metastasis. Subjects treated with adjuvant ADT who developed metastasis have higher ARS scores compared to non-metastatic subjects or subjects that were not treated with adjuvant hormones.
FIGS. 4A and 4B show interaction plots between a prognostic model (RF22) and a predictive model (ARS) in the training cohort. FIG. 4A shows an interaction plot of a prognostic model (RF22) for reference. The RF22 gives high scores for metastatic subjects regardless of their treatment. FIG. 4B shows an interaction plot showing the ARS, a predictive model that has a significant interaction with treatment. The ARS gives higher scores for subjects who metastasize and received treatment.
FIG. 5 shows an ADT signature representing multiple biological pathways involved in cancer progression and neuronal development.
FIG. 6 shows model validation in independent cohorts showing subjects failing ADT are at higher risk of metastasis. The ARS score distribution in independent validation cohort. The ARS produces higher scores for ADT resistant subjects and base line scores for subjects responding to ADT or those who did not receive ADT.
FIGS. 7A and 7B show survival analysis of the ARS model. Kaplan Meier curve showing survival differences between treated and untreated subjects with (FIG. 7A) low ARS scores (p-value of 0.086) and (FIG. 7B) high ARS scores (p-value < 0.001) (Bonferroni adjusted log-rank)). Low and high ARS scores defined by median.
FIG. 8 shows a prediction curve of ARS and post-RP survival. Prediction curve at 10 years post-RP showing treated subjects with high scores are more likely to develop metastasis.
FIG. 9 shows model validation in independent natural history cohort with no ADT treatment. The ARS score distributions in independent validation cohort of untreated subjects. The figure shows that untreated subjects receive baseline scores whether they metastasize or not.
FIG. 10 shows the ARS prediction of rapid castration resistant prostate cancer (CRPC) after ADT. In the CRPC subjects of the natural history cohort, subjects who developed metastasis and received subsequent ADT tended to fail treatment faster if they had high ARS scores (p=0.07).
FIG. 11 shows the ARS in small cell (SC) carcinoma. Subjects with small cell carcinoma that are resistant to ADT are classified by the model to be resistant to hormone therapy.
FIG. 12 shows the Genomic Paradigm for Enhanced Decision-Making. The ARS model will be implemented in the clinic as part of the Decipher test. Subjects with high Decipher scores are at higher risk of developing metastasis and require a second line of therapy post-surgery.
FIG. 13 shows a study diagram with training and validation cohorts and metastatic outcome.
FIG. 14 shows an exemplary flowchart for developing an ARS model of the invention.
FIG. 15 shows cumulative incidences of metastasis stratified by ADT treatment status among (A) patients with Low ARS scores and (B) patients with High ARS scores.
FIG. 16 shows risk-adjusted 10-year prediction curves based on multivariable analysis results from Validation Set II.

### DETAILED DESCRIPTION OF THE INVENTION

The practice of the present invention will employ, unless otherwise indicated, conventional methods of medicine, biochemistry, molecular biology and recombinant DNA techniques, within the skill of the art. Such techniques are explained fully in the literature. See, e.g., Prostate Cancer: Science and Clinical Practice (J.H. Mydlo and C.J. Godec eds., Academic Press, 2nd edition, 2015); Prostate Cancer: Biochemistry, Molecular Biology and Genetics (Protein Reviews 16, D.J. Tindall ed., Springer, 2013); A.L. Lehninger, Biochemistry (Worth Publishers, Inc., current addition); Sambrook, et al., Molecular Cloning: A Laboratory Manual (3rd Edition, 2001); and Methods In Enzymology (S. Colowick and N. Kaplan eds., Academic Press, Inc.).

### DEFINITIONS

In describing the present invention, the following terms will be employed, and are intended to be defined as indicated below.

It must be noted that, as used in this specification and the appended claims, the singular forms "a," "an" and "the" include plural referents unless the content clearly dictates otherwise. Thus, for example, reference to "a nucleic acid" includes a mixture of two or more such nucleic acids, and the like.

By "resistant to ADT" is meant, when referring to a subject that has prostate cancer, that the condition of the subject does not improve with ADT. Subjects who are resistant to ADT may experience tumor growth or cancer metastasis after ADT treatment and reduced survival time compared to subjects who respond to ADT.

The term "survival" as used herein means the time from the start of ADT to the time of death.

A "biomarker" in the context of the present invention refers to a biological compound, such as a polynucleotide or polypeptide which is differentially expressed in a sample taken from subjects having prostate cancer who are resistant to ADT as compared to a comparable sample taken from control subjects (e.g., subjects who are not resistant to ADT). The biomarker can be a nucleic acid, a fragment of a nucleic acid, a polynucleotide, or an oligonucleotide that can be detected and/or quantified. Biomarkers include nucleic acids comprising nucleotide sequences from genes or RNA transcripts of genes, including but not limited to, DAND5, GABRB3, KLK12, NAV2, POGK, RIMS2, SNCAIP, TET1, TMEM176A, ELL2, ADAMTS14, CDKN2C, KCNMB2, MUC1, PLEKHH2, TNIK, AGTR1, BAG3, C10orf81, C8orf4, CRISP2, GALM, GHR, POLD4, RRAS, SPATA13, TMEFF2, ARHGAP11A, ASB16, CASP2, FAM57B, FOXM1, GNAZ, AC084018.1, NAV1, RRM2, TFAP4, HEPN1, KIF1C, MPDU1, RLN1, SELE, WDR93, ATF5, HEY2, NUP210, CDH3, CREB3L1, SLC35F2, TRPV6, FAM134A, NSMCE4A, SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12, NLRP13, MPP7, CFL1, DESI2, OR51E2, KCNMB1, DLGAP1, SPRR1A, CROT, KIFC1, POLD4, CASP2, WHSC1, MPZL2, NAV1, RNF168, FOXM1, ZC3H11A, FAM3D, KCNK17, PLXNA2, SUOX, ANP32E, REST, NKX2.2, RBBP8, NSMCE4A, H19, ATP1A2, PLXNC1, NUP62, ACAA2, ADH1C, THYN1, COX7A2L and MAP1B and their expression products.

A "similarity value" is a number that represents the degree of similarity between two things being compared. For example, a similarity value may be a number that indicates the overall similarity between a subject's expression profile using specific phenotype-related biomarkers and reference value ranges for the biomarkers in one or more control samples or a reference expression profile (e.g., the similarity to an "ADT non-responder" expression profile or an "ADT responder" expression profile). The similarity value may be expressed as a similarity metric, such as a correlation coefficient, or may simply be expressed as the expression level difference, or the aggregate of the expression level differences, between levels of biomarkers in a subject sample and a control sample or reference expression profile.

The terms "tumor," "cancer" and "neoplasia" are used interchangeably and refer to a cell or population of cells whose growth, proliferation or survival is greater than growth, proliferation or survival of a normal counterpart cell, e.g. a cell proliferative, hyperproliferative or differentiative disorder. Typically, the growth is uncontrolled. The term "malignancy" refers to invasion of nearby tissue. The term "metastasis" or a secondary, recurring or recurrent tumor, cancer or neoplasia refers to spread or dissemination of a tumor, cancer or neoplasia to other sites, locations or regions within the subject, in which the sites, locations or regions are distinct from the primary tumor or cancer. Neoplasia, tumors and cancers include benign, malignant, metastatic and non-metastatic types, and include any stage (I, II, III, IV or V) or grade (G1, G2, G3, etc.) of neoplasia, tumor, or cancer, or a neoplasia, tumor, cancer or metastasis that is progressing, worsening, stabilized or in remission. In particular, the terms "tumor," "cancer" and "neoplasia" include carcinomas, such as squamous cell carcinoma, adenocarcinoma, adenosquamous carcinoma, anaplastic carcinoma, large cell carcinoma, and small cell carcinoma.

The term "derived from" is used herein to identify the original source of a molecule but is not meant to limit the method by which the molecule is made which can be, for example, by chemical synthesis or recombinant means.

"Recombinant" as used herein to describe a nucleic acid molecule means a polynucleotide of genomic, cDNA, viral, semisynthetic, or synthetic origin which, by virtue of its origin or manipulation is not associated with all or a portion of the polynucleotide with which it is associated in nature. The term "recombinant" as used with respect to a protein or polypeptide means a polypeptide produced by expression of a recombinant polynucleotide. In general, the gene of interest is cloned and then expressed in transformed organisms, as described further below. The host organism expresses the foreign gene to produce the protein under expression conditions.

"Substantially purified" generally refers to isolation of a substance (compound, polynucleotide, oligonucleotide, protein, or polypeptide) such that the substance comprises the majority percent of the sample in which it resides. Typically in a sample, a substantially purified component comprises 50%, preferably 80%-85%, more preferably 90-95% of the sample. Techniques for purifying polynucleotides oligonucleotides and polypeptides of interest are well-known in the art and include, for example, ion-exchange chromatography, affinity chromatography and sedimentation according to density.

By "isolated" is meant, when referring to a polypeptide, that the indicated molecule is separate and discrete from the whole organism with which the molecule is found in nature or is present in the substantial absence of other biological macro molecules of the same type. The term "isolated" with respect to a polynucleotide or oligonucleotide is a nucleic acid molecule devoid, in whole or part, of sequences normally associated with it in nature; or a sequence, as it exists in nature, but having heterologous sequences in association therewith; or a molecule disassociated from the chromosome.

The terms "polynucleotide," "oligonucleotide," "nucleic acid" and "nucleic acid molecule" are used herein to include a polymeric form of nucleotides of any length, either ribonucleotides or deoxyribonucleotides. This term refers only to the primary structure of the molecule. Thus, the term includes triple-, double- and single-stranded DNA, as well as triple-, double- and single-stranded RNA. It also includes modifications, such as by methylation and/or by capping, and unmodified forms of the polynucleotide. More particularly, the terms "polynucleotide," "oligonucleotide," "nucleic acid" and "nucleic acid molecule" include polydeoxyribonucleotides (containing 2-deoxy-D-ribose), polyribonucleotides (containing D-ribose), any other type of polynucleotide which is an N- or C-glycoside of a purine or pyrimidine base, and other polymers containing nonnucleotidic backbones, for example, polyamide (e.g., peptide nucleic acids (PNAs)) and polymorpholino (commercially available from the Anti-Virals, Inc., Corvallis, Oregon, as Neugene) polymers, and other synthetic sequence-specific nucleic acid polymers providing that the polymers contain nucleobases in a configuration which allows for base pairing and base stacking, such as is found in DNA and RNA. There is no intended distinction in length between the terms "polynucleotide," "oligonucleotide," "nucleic acid" and "nucleic acid molecule," and these terms will be used interchangeably. Thus, these terms include, for example, 3'-deoxy-2',5'-DNA, oligodeoxyribonucleotide N3' P5' phosphoramidates, 2'-O-alkyl-substituted RNA, double- and single-stranded DNA, as well as double- and single-stranded RNA, DNA:RNA hybrids, and hybrids between PNAs and DNA or RNA, and also include known types of modifications, for example, labels which are known in the art, methylation, "caps," substitution of one or more of the naturally occurring nucleotides with an analog, internucleotide modifications such as, for example, those with uncharged linkages (e.g., methyl phosphonates, phosphotriesters, phosphoramidates, carbamates, etc.), with negatively charged linkages (e.g., phosphorothioates, phosphorodithioates, etc.), and with positively charged linkages (e.g., aminoalklyphosphoramidates, aminoalkylphosphotriesters), those containing pendant moieties, such as, for example, proteins (including nucleases, toxins, antibodies, signal peptides, poly-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide or oligonucleotide. The term also includes locked nucleic acids (e.g., comprising a ribonucleotide that has a methylene bridge between the 2'-oxygen atom and the 4'-carbon atom). See, for example, Kurreck et al. (2002) Nucleic Acids Res. 30: 1911-1918; Elayadi et al. (2001) Curr. Opinion Invest. Drugs 2: 558-561; Orum et al. (2001) Curr. Opinion Mol. Ther. 3: 239-243; Koshkin et al. (1998) Tetrahedron 54: 3607-3630; Obika et al. (1998) Tetrahedron Lett. 39: 5401-5404.

As used herein, the term "probe" or "oligonucleotide probe" refers to a polynucleotide, as defined above, that contains a nucleic acid sequence complementary to a nucleic acid sequence present in the target nucleic acid analyte (e.g., biomarker). The polynucleotide regions of probes may be composed of DNA, and/or RNA, and/or synthetic nucleotide analogs. Probes may be labeled in order to detect the target sequence. Such a label may be present at the 5' end, at the 3' end, at both the 5' and 3' ends, and/or internally.

The term "amplicon" refers to the amplified nucleic acid product of a PCR reaction or other nucleic acid amplification process (*e.g*., ligase chain reaction (LGR), nucleic acid sequence based amplification (NASBA), transcription-mediated amplification (TMA), Q-beta amplification, strand displacement amplification, or target mediated amplification). Amplicons may comprise RNA or DNA depending on the technique used for amplification.

The terms "hybridize" and "hybridization" refer to the formation of complexes between nucleotide sequences which are sufficiently complementary to form complexes via Watson-Crick base pairing.

It will be appreciated that the hybridizing sequences need not have perfect complementarity to provide stable hybrids. In many situations, stable hybrids will form where fewer than about 10% of the bases are mismatches, ignoring loops of four or more nucleotides. Accordingly, as used herein the term "complementary" refers to an oligonucleotide that forms a stable duplex with its "complement" under assay conditions, generally where there is about 90% or greater homology.

The terms "selectively detects" or "selectively detecting" refer to the detection of biomarker nucleic acids using oligonucleotides, e.g., primers or probes that are capable of detecting a particular biomarker nucleic acid, for example, by amplifying and/or binding to at least a portion of the biomarker nucleic acid, but do not amplify and/or bind to sequences from other nucleic acids under appropriate hybridization conditions.

As used herein, the terms "label" and "detectable label" refer to a molecule capable of detection, including, but not limited to, radioactive isotopes, fluorescers, chemiluminescers, chromophores, enzymes, enzyme substrates, enzyme cofactors, enzyme inhibitors, semiconductor nanoparticles, dyes, metal ions, metal sols, ligands (e.g., biotin, streptavidin or haptens) and the like. The term "fluorescer" refers to a substance or a portion thereof which is capable of exhibiting fluorescence in the detectable range. Particular examples of labels which may be used in the practice of the invention include, but are not limited to, horseradish peroxidase (HRP), SYBR® green, SYBR® gold, fluorescein, carboxyfluorescein (FAM), Alexa Fluor dyes, Cy3, Cy5, Cy7, 7-amino-4-methylcoumarin-3-acetic acid (AMCA), 5-(and-6)-carboxy-X-rhodamine, lissamine rhodamine, fluorescein-5-isothiocyanate (FITC), 7-diethylaminocoumarin-3-carboxylic acid, tetramethylrhodamine-5 -(and-6)-isothiocyanate, 5-(and-6)-carboxytetramethylrhodamine, 7-hydroxycoumarin-3-carboxylic acid, 6-[fluorescein 5-(and-6)-carboxamido]hexanoic acid, N-(4,4-difluoro-5,7-dimethyl-4-bora-3a,4a diaza-3-indacenepropionic acid, eosin-5-isothiocyanate; erythrosine-5-isothiocyanate, 5-(and-6)-carboxyrhodamine 6G, CASCADE blue aectylazide, CAL Fluor Orange 560, CAL Fluor Red 610, Quasar Blue 670, tetramethyl rhodamine (TAMRA), 2', 4', 5', 7'- tetrachloro-4-7-dichlorofluorescein (TET), rhodamine, dansyl, umbelliferone, dimethyl acridinium ester (DMAE), Texas red, Pacific Blue, Pacific Orange, quantum dots, luminol, NADPH, and α-β-galactosidase.

The terms "subject," "individual," and "subject," are used interchangeably herein and refer to any mammalian subject, particularly humans. Other subjects may include cattle, dogs, cats, guinea pigs, rabbits, rats, mice, horses, and so on. In some cases, the methods of the invention find use in experimental animals, in veterinary application, and in the development of animal models, including, but not limited to, rodents including mice, rats, and hamsters; and primates.

Before describing the present invention in detail, it is to be understood that this invention is not limited to particular formulations or process parameters as such may, of course, vary. It is also to be understood that the terminology used herein is for the purpose of describing particular embodiments of the invention only, and is not intended to be limiting.

Although a number of methods and materials similar or equivalent to those described herein can be used in the practice of the present invention, the preferred materials and methods are described herein.

The present invention is based on the discovery of biomarkers for identifying individuals who will be resistant to androgen deprivation therapy for treatment of prostate cancer. In particular, the invention relates to an androgen deprivation therapy resistance signature based on expression levels of genes that are differentially expressed between responders and non-responders to androgen deprivation therapy and its use in identifying individuals likely to be resistant to androgen deprivation therapy, who are in need of treatment for prostate cancer by other methods.

In order to further an understanding of the invention, a more detailed discussion is provided below regarding the identified ADT resistance signature and methods of screening and treating subjects for prostate cancer.

### Biomarkers

Biomarkers that can be used in the practice of the invention include nucleic acids comprising nucleotide sequences from genes or RNA transcripts of genes, including but not limited to, DAND5, GABRB3, KLK12, NAV2, POGK, RIMS2, SNCAIP, TET1, TMEM176A, ELL2, ADAMTS14, CDKN2C, KCNMB2, MUC1, PLEKHH2, TNIK, AGTR1, BAG3, C10orf81, C8orf4, CRISP2, GALM, GHR, POLD4, RRAS, SPATA13, TMEFF2, ARHGAP11A, ASB16, CASP2, FAM57B, FOXM1, GNAZ, AC084018.1, NAV1, RRM2, TFAP4, HEPN1, KIF1C, MPDU1, RLN1, SELE, WDR93, ATF5, HEY2, NUP210, CDH3, CREB3L1, SLC35F2, TRPV6, FAM134A, NSMCE4A, SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12, NLRP13, MPP7, CFL1, DESI2, OR51E2, KCNMB1, DLGAP1, SPRR1A, CROT, KIFC1, POLD4, CASP2, WHSC1, MPZL2, NAV1, RNF168, FOXM1, ZC3H11A, FAM3D, KCNK17, PLXNA2, SUOX, ANP32E, REST, NKX2.2, RBBP8, NSMCE4A, H19, ATP1A2, PLXNC1, NUP62, ACAA2, ADH1C, THYN1, COX7A2L and MAP1B and their expression products. Expression profiles of these biomarkers are useful for determining whether an individual who has prostate cancer is likely to be resistant or respond to treatment with ADT.

Accordingly, in one aspect, the invention provides a method for predicting resistance to androgen deprivation therapy (ADT) for a subject who has prostate cancer comprising measuring the level of a plurality of biomarkers in a biological sample comprising cancer cells derived from the subject, and analyzing the levels of the biomarkers and comparing with respective reference value ranges for the biomarkers for subjects who are resistant to ADT (e.g., "ADT non-responder" expression profile) and subjects who are not resistant to ADT ("ADT responder" expression profile), wherein similarity to an "ADT non-responder" expression profile indicates that the subject is resistant to ADT.

Additionally, a subject may also be identified as resistant to ADT based on an ADT resistance signature (ARS) score, calculated based on the level of expression of the plurality of biomarkers (see Examples), wherein a higher ARS score for the subject compared to reference value ranges for a control subject (i.e., subject that is not resistant to ADT) indicates that the subject is resistant to ADT.

In certain embodiments, a panel of biomarkers is used for evaluating a subject who has prostate cancer for resistance to ADT. Biomarker panels typically comprise at least 3 biomarkers and up to 60 biomarkers, including any number of biomarkers in between, such as 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50, 55, or 60 biomarkers. Described herein is a biomarker panel comprising at least 3, or at least 4, or at least 5, or at least 6, or at least 7, or at least 8, or at least 9, or at least 10, or at least 15, at least 20, at least 25, at least 30, at least 35, at least 40, at least 45, at least 50, or at least 55 or more biomarkers. Although smaller biomarker panels are usually more economical, larger biomarker panels (i.e., greater than 30 biomarkers) have the advantage of providing more detailed information and can also be used in the practice of the invention.

Described herein is a panel of biomarkers comprising one or more nucleic acids comprising a nucleotide sequence from a gene or an RNA transcript of a gene selected from SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12, NLRP13, MPP7, CFL1, DESI2, OR51E2, KCNMB1, DLGAP1, SPRR1A, CROT, KIFC1, POLD4, CASP2, WHSC1, MPZL2, NAV1, RNF168, FOXM1, ZC3H11A, FAM3D, KCNK17, PLXNA2, SUOX, ANP32E, REST, NKX2.2, RBBP8, NSMCE4A, H19, ATP1A2, PLXNC1, NUP62, ACAA2, ADH1C, THYN1, COX7A2L, MAP1B or a combination thereof.

Described herein is a panel of biomarkers comprises nucleic acids comprising gene sequences of SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12, NLRP13 or s combination thereof.

Described herein is a panel of biomarkers comprising one or more nucleic acids comprising a nucleotide sequence from a gene or an RNA transcript of a gene selected from DAND5, GABRB3, KLK12, NAV2, POGK, RIMS2, SNCAIP, TET1, TMEM176A, ELL2, ADAMTS14, CDKN2C, KCNMB2, MUC1, PLEKHH2, TNIK, AGTR1, BAG3, C10orf81, C8orf4, CRISP2, GALM, GHR, POLD4, RRAS, SPATA13, TMEFF2, ARHGAP11A, ASB16, CASP2, FAM57B, FOXM1, GNAZ, AC084018.1, NAV1, RRM2, TFAP4, HEPN1, KIF1C, MPDU1, RLN1, SELE, WDR93, ATF5, HEY2, NUP210, CDH3, CREB3L1, SLC35F2, TRPV6, FAM134A, NSMCE4A or a combination thereof.

Described herein is a panel of biomarkers comprises nucleic acids comprising gene sequences of DAND5, GABRB3, RIMS2, SNCAIP, TMEM176A, KCNMB2, PLEKHH2, AGTR1, BAG3, C10orf81, C8orf4, CRISP2, GALM, GHR, RRAS, SPATA13, TMEFF2, ARHGAP11A, GNAZ, AC084018.1, RRM2, TFAP4, HEPN1, MPDU1, RLN1, SELE, WDR93, ATF5, HEY2, CREB3L1, SLC35F2, FAM134A, NSMCE4A or a combination thereof. Described herein, the panel of biomarkers further comprises one or more nucleic acids comprising gene sequences of KLK12, NAV2, POGK, TET1, ELL2, ADAMTS14, CDKN2C, MUC1, TNIK, POLD4, ASB16, CASP2, FAM57B, FOXM1, NAV1, KIF1C, NUP210, CDH3, TRPV6 or a combination thereof.

Described herein, the panel of biomarkers comprises nucleic acids comprising gene sequences of SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12, NLRP13, MPP7, CFL1, DESI2, OR51E2, KCNMB1, DLGAP1, SPRR1A, CROT, KIFC1, POLD4, CASP2, WHSC1, MPZL2, NAV1, RNF168, FOXM1, ZC3H11A, FAM3D, KCNK17, PLXNA2, SUOX, ANP32E, REST, NKX2.2, RBBP8, NSMCE4A, H19, ATP1A2, PLXNC1, NUP62, ACAA2, ADH1C, THYN1, COX7A2L, MAP1B or a combination thereof.

Described herein, the panel of biomarkers comprises nucleic acids comprising gene sequences of DAND5, GABRB3, KLK12, NAV2, POGK, RIMS2, SNCAIP, TET1, TMEM176A, ELL2, ADAMTS14, CDKN2C, KCNMB2, MUC1, PLEKHH2, TNIK, AGTR1, BAG3, C10orf81, C8orf4, CRISP2, GALM, GHR, POLD4, RRAS, SPATA13, TMEFF2, ARHGAP11A, ASB16, CASP2, FAM57B, FOXM1, GNAZ, AC084018.1, NAV1, RRM2, TFAP4, HEPN1, KIF1C, MPDU1, RLN1, SELE, WDR93, ATF5, HEY2, NUP210, CDH3, CREB3L1, SLC35F2, TRPV6, FAM134A, NSMCE4A or a combination thereof.

The methods described herein may be used to determine if a subject should be treated for prostate cancer with ADT or some other method. For example, a subject is selected for treatment for prostate cancer with ADT if the subject is not identified as being resistant to ADT based on a biomarker expression profile or an ARS score, as described herein. On the contrary, a subject identified as resistant to ADT, based on a biomarker expression profile or an ARS score, is not treated with ADT, but rather, with some other cancer therapy, such as, but not limited to, surgery, radiation therapy, chemotherapy, immunotherapy, or biologic therapy, or any combination thereof.

It is understood that the biomarkers in a sample can be measured by any suitable method known in the art. Measurement of the expression level of a biomarker can be direct or indirect. For example, the abundance levels of RNAs or proteins can be directly quantitated. Alternatively, the amount of a biomarker can be determined indirectly by measuring abundance levels of cDNAs, amplified RNAs or DNAs, or by measuring quantities or activities of RNAs, proteins, or other molecules (e.g., metabolites) that are indicative of the expression level of the biomarker.

The levels of transcripts of specific biomarker genes can be determined from the amount of mRNA, or polynucleotides derived therefrom, present in a biological sample. Polynucleotides can be detected and quantitated by a variety of methods including, but not limited to, microarray analysis, polymerase chain reaction (PCR), reverse transcriptase polymerase chain reaction (RT-PCR), Northern blot, and serial analysis of gene expression (SAGE). See, e.g., Draghici Data Analysis Tools for DNA Microarrays, Chapman and Hall/CRC, 2003; Simon et al. Design and Analysis of DNA Microarray Investigations, Springer, 2004; Real-Time PCR: Current Technology and Applications, Logan, Edwards, and Saunders eds., Caister Academic Press, 2009; Bustin A-Z of Quantitative PCR (IUL Biotechnology, No. 5), International University Line, 2004; Velculescu et al. (1995) Science 270: 484-487; Matsumura et al. (2005) Cell. Microbiol. 7: 11-18; Serial Analysis of Gene Expression (SAGE): Methods and Protocols (Methods in Molecular Biology), Humana Press, 2008.

In certain embodiments, nucleic acids from a biological sample are isolated, purified, and/or amplified prior to analysis using methods well-known in the art. See, e.g., Green and Sambrook Molecular Cloning: A Laboratory Manual (Cold Spring Harbor Laboratory Press; 4th edition, 2012); and Current Protocols in Molecular Biology (Ausubel ed., John Wiley & Sons, 1995).

### Targets

In some instances, assaying the expression level of a plurality of biomarker genes comprises detecting and/or quantifying a plurality of target nucleic acid analytes. In some embodiments, assaying the expression level of a plurality of biomarker genes comprises sequencing a plurality of target nucleic acids. In some embodiments, assaying the expression level of a plurality of biomarker genes comprises amplifying a plurality of target nucleic acids. In some embodiments, assaying the expression level of a plurality of biomarker genes comprises conducting a multiplexed reaction on a plurality of target analytes.

The methods disclosed herein often comprise assaying the expression level of a plurality of targets. The plurality of targets may comprise coding targets and/or non-coding targets of a protein-coding gene or a non protein-coding gene. A protein-coding gene structure may comprise an exon and an intron. The exon may further comprise a coding sequence (CDS) and an untranslated region (UTR). The protein-coding gene may be transcribed to produce a pre-mRNA and the pre-mRNA may be processed to produce a mature mRNA. The mature mRNA may be translated to produce a protein.

A non protein-coding gene structure may comprise an exon and intron. Usually, the exon region of a non protein-coding gene primarily contains a UTR. The non protein-coding gene may be transcribed to produce a pre-mRNA and the pre-mRNA may be processed to produce a non-coding RNA (ncRNA).

A coding target may comprise a coding sequence of an exon. A non-coding target may comprise a UTR sequence of an exon, intron sequence, intergenic sequence, promoter sequence, non-coding transcript, CDS antisense, intronic antisense, UTR antisense, or non-coding transcript antisense. A non-coding transcript may comprise a non-coding RNA (ncRNA).

In some instances, the plurality of targets comprises one or more targets selected from Table 1 or Table 4. In some instances, the plurality of targets comprises at least about 2, at least about 3, at least about 4, at least about 5, at least about 6, at least about 7, at least about 8, at least about 9, at least about 10, at least about 15, at least about 20, at least about 25, at least about 30, at least about 35, at least about 40, at least about 45, or at least about 50 targets selected from Table 1 or Table 4.

In some instances, the plurality of targets comprises a coding target, non-coding target, or any combination thereof. In some instances, the coding target comprises an exonic sequence. In other instances, the non-coding target comprises a non-exonic or exonic sequence. Alternatively, a non-coding target comprises a UTR sequence, an intronic sequence, antisense, or a non-coding RNA transcript. In some instances, a non-coding target comprises sequences which partially overlap with a UTR sequence or an intronic sequence. A non-coding target also includes non-exonic and/or exonic transcripts. Exonic sequences may comprise regions on a protein-coding gene, such as an exon, UTR, or a portion thereof. Non-exonic sequences may comprise regions on a protein-coding, non protein-coding gene, or a portion thereof. For example, non-exonic sequences may comprise intronic regions, promoter regions, intergenic regions, a non-coding transcript, an exon anti-sense region, an intronic anti-sense region, UTR anti-sense region, non-coding transcript anti-sense region, or a portion thereof. In other instances, the plurality of targets comprises a non-coding RNA transcript.

The plurality of targets may comprise one or more targets selected from a classifier disclosed herein. The classifier may be generated from one or more models or algorithms. The one or more models or algorithms may be Naive Bayes (NB), recursive Partitioning (Rpart), random forest (RF), support vector machine (SVM), k-nearest neighbor (KNN), high dimensional discriminate analysis (HDDA), or a combination thereof. The classifier may have an AUC of equal to or greater than 0.60. The classifier may have an AUC of equal to or greater than 0.61. The classifier may have an AUC of equal to or greater than 0.62. The classifier may have an AUC of equal to or greater than 0.63. The classifier may have an AUC of equal to or greater than 0.64. The classifier may have an AUC of equal to or greater than 0.65. The classifier may have an AUC of equal to or greater than 0.66. The classifier may have an AUC of equal to or greater than 0.67. The classifier may have an AUC of equal to or greater than 0.68. The classifier may have an AUC of equal to or greater than 0.69. The classifier may have an AUC of equal to or greater than 0.70. The classifier may have an AUC of equal to or greater than 0.75. The classifier may have an AUC of equal to or greater than 0.77. The classifier may have an AUC of equal to or greater than 0.78. The classifier may have an AUC of equal to or greater than 0.79. The classifier may have an AUC of equal to or greater than 0.80. The AUC may be clinically significant based on its 95% confidence interval (CI). The accuracy of the classifier may be at least about 70%. The accuracy of the classifier may be at least about 73%. The accuracy of the classifier may be at least about 75%. The accuracy of the classifier may be at least about 77%. The accuracy of the classifier may be at least about 80%. The accuracy of the classifier may be at least about 83%. The accuracy of the classifier may be at least about 84%. The accuracy of the classifier may be at least about 86%. The accuracy of the classifier may be at least about 88%. The accuracy of the classifier may be at least about 90%. The p-value of the classifier may be less than or equal to 0.05. The p-value of the classifier may be less than or equal to 0.04. The p-value of the classifier may be less than or equal to 0.03. The p-value of the classifier may be less than or equal to 0.02. The p-value of the classifier may be less than or equal to 0.01. The p-value of the classifier may be less than or equal to 0.008. The p-value of the classifier may be less than or equal to 0.006. The p-value of the classifier may be less than or equal to 0.004. The p-value of the classifier may be less than or equal to 0.002. The p-value of the classifier may be less than or equal to 0.001.

The plurality of targets may comprise one or more targets selected from a Random Forest (RF) classifier. The plurality of targets may comprise two or more targets selected from a Random Forest (RF) classifier. The plurality of targets may comprise three or more targets selected from a Random Forest (RF) classifier. The plurality of targets may comprise 2, 3, 4, 5, 6, 7, 8, 9, 10, 11, 12, 13, 14, 15, 20, 25, 30, 35, 40, 45, 50, or more targets selected from a Random Forest (RF) classifier. The RF classifier may be an RF2, and RF3, or an RF4 classifier. The RF classifier may be an RF22 classifier (e.g., a Random Forest classifier with 22 targets).

### Probes/Primers

Described herein is a probe set for diagnosing, monitoring and/or predicting a status or outcome of a prostate cancer in a subject comprising a plurality of probes, wherein (i) the probes in the set are capable of detecting an expression level of at least one target selected from Table 1 or Table 4; and (ii) the expression level determines the cancer status of the subject with at least about 40% specificity.

The probe set may comprise one or more polynucleotide probes. Individual polynucleotide probes comprise a nucleotide sequence derived from the nucleotide sequence of the target sequences or complementary sequences thereof. The nucleotide sequence of the polynucleotide probe is designed such that it corresponds to, or is complementary to the target sequences. The polynucleotide probe can specifically hybridize under either stringent or lowered stringency hybridization conditions to a region of the target sequences, to the complement thereof, or to a nucleic acid sequence (such as a cDNA) derived therefrom.

The selection of the polynucleotide probe sequences and determination of their uniqueness may be carried out *in silico* using techniques known in the art, for example, based on a BLASTN search of the polynucleotide sequence in question against gene sequence databases, such as the Human Genome Sequence, UniGene, dbEST or the non-redundant database at NCBI. Described herein, the polynucleotide probe is complementary to a region of a target mRNA derived from a target sequence in the probe set. Computer programs can also be employed to select probe sequences that may not cross hybridize or may not hybridize non-specifically.

In some instances, microarray hybridization of RNA, extracted from prostate cancer tissue samples and amplified, may yield a dataset that is then summarized and normalized by the fRMA technique. After removal (or filtration) of cross-hybridizing PSRs, and PSRs containing less than 4 probes, the remaining PSRs can be used in further analysis. Following fRMA and filtration, the data can be decomposed into its principal components and an analysis of variance model is used to determine the extent to which a batch effect remains present in the first 10 principal components.

These remaining PSRs can then be subjected to filtration by a T-test between CR (clinical recurrence) and non-CR samples. Using a p-value cut-off of 0.01, the remaining features (e.g., PSRs) can be further refined. Feature selection can be performed by regularized logistic regression using the elastic-net penalty. The regularized regression may be bootstrapped over 1000 times using all training data; with each iteration of bootstrapping, features that have non-zero co-efficient following 3-fold cross validation can be tabulated. In some instances, features that were selected in at least 25% of the total runs were used for model building.

The polynucleotide probes described herein may range in length from about 15 nucleotides to the full length of the coding target or non-coding target. Described herein, the polynucleotide probes are at least about 15 nucleotides in length. Described herein, the polynucleotide probes are at least about 20 nucleotides in length. Described herein, the polynucleotide probes are at least about 25 nucleotides in length. Described herein, the polynucleotide probes are between about 15 nucleotides and about 500 nucleotides in length. Described herein, the polynucleotide probes are between about 15 nucleotides and about 450 nucleotides, about 15 nucleotides and about 400 nucleotides, about 15 nucleotides and about 350 nucleotides, about 15 nucleotides and about 300 nucleotides, about 15 nucleotides and about 250 nucleotides, about 15 nucleotides and about 200 nucleotides in length. Described herein, the probes are at least 15 nucleotides in length. Described herein, the probes are at least 15 nucleotides in length. Described herein, the probes are at least 20 nucleotides, at least 25 nucleotides, at least 50 nucleotides, at least 75 nucleotides, at least 100 nucleotides, at least 125 nucleotides, at least 150 nucleotides, at least 200 nucleotides, at least 225 nucleotides, at least 250 nucleotides, at least 275 nucleotides, at least 300 nucleotides, at least 325 nucleotides, at least 350 nucleotides, at least 375 nucleotides in length.

The polynucleotide probes of a probe set can comprise RNA, DNA, RNA or DNA mimetics, or combinations thereof, and can be single-stranded or double-stranded. Thus the polynucleotide probes can be composed of naturally-occurring nucleobases, sugars and covalent internucleoside (backbone) linkages as well as polynucleotide probes having non-naturally-occurring portions which function similarly. Such modified or substituted polynucleotide probes may provide desirable properties such as, for example, enhanced affinity for a target gene and increased stability. The probe set may comprise a coding target and/or a non-coding target. Preferably, the probe set comprises a combination of a coding target and non-coding target.

Described herein, the probe set comprise a plurality of target sequences that hybridize to at least about 5 coding targets and/or non-coding targets selected from Table 1 or Table 4. Alternatively, the probe set comprise a plurality of target sequences that hybridize to at least about 10 coding targets and/or non-coding targets selected from Table 1 or Table 4. Described herein, the probe set comprise a plurality of target sequences that hybridize to at least about 15 coding targets and/or non-coding targets selected from Table 1 or Table 4. Described herein, the probe set comprise a plurality of target sequences that hybridize to at least about 20 coding targets and/or non-coding targets selected from Table 1 or Table 4. Described herein, the probe set comprise a plurality of target sequences that hybridize to at least about 30 coding targets and/or non-coding targets selected from Table 1 or Table 4.

The system described herein further provides for primers and primer pairs capable of amplifying target sequences defined by the probe set, or fragments or subsequences or complements thereof. The nucleotide sequences of the probe set may be provided in computer-readable media for *in silico* applications and as a basis for the design of appropriate primers for amplification of one or more target sequences of the probe set.

Primers based on the nucleotide sequences of target sequences can be designed for use in amplification of the target sequences. For use in amplification reactions such as PCR, a pair of primers can be used. The exact composition of the primer sequences is not critical , but for most applications the primers may hybridize to specific sequences of the probe set under stringent conditions, particularly under conditions of high stringency, as known in the art. The pairs of primers are usually chosen so as to generate an amplification product of at least about 50 nucleotides, more usually at least about 100 nucleotides. Algorithms for the selection of primer sequences are generally known, and are available in commercial software packages. These primers may be used in standard quantitative or qualitative PCR-based assays to assess transcript expression levels of RNAs defined by the probe set. Alternatively, these primers may be used in combination with probes, such as molecular beacons in amplifications using real-time PCR.

Described herein, the primers or primer pairs, when used in an amplification reaction, specifically amplify at least a portion of a nucleic acid sequence of a target selected from Table 1 or Table 4 (or subgroups thereof as set forth herein), an RNA form thereof, or a complement to either thereof.

A label can optionally be attached to or incorporated into a probe or primer polynucleotide to allow detection and/or quantitation of a target polynucleotide representing the target sequence of interest. The target polynucleotide may be the expressed target sequence RNA itself, a cDNA copy thereof, or an amplification product derived therefrom, and may be the positive or negative strand, so long as it can be specifically detected in the assay being used. Similarly, an antibody may be labeled.

In certain multiplex formats, labels used for detecting different targets may be distinguishable. The label can be attached directly (e.g., via covalent linkage) or indirectly, e.g., via a bridging molecule or series of molecules (e.g., a molecule or complex that can bind to an assay component, or via members of a binding pair that can be incorporated into assay components, e.g. biotin-avidin or streptavidin). Many labels are commercially available in activated forms which can readily be used for such conjugation (for example through amine acylation), or labels may be attached through known or determinable conjugation schemes, many of which are known in the art.

Labels described herein include any substance which can be detected when bound to or incorporated into the biomolecule of interest. Any effective detection method can be used, including optical, spectroscopic, electrical, piezoelectrical, magnetic, Raman scattering, surface plasmon resonance, colorimetric, calorimetric, etc. A label is typically selected from a chromophore, a lumiphore, a fluorophore, one member of a quenching system, a chromogen, a hapten, an antigen, a magnetic particle, a material exhibiting nonlinear optics, a semiconductor nanocrystal, a metal nanoparticle, an enzyme, an antibody or binding portion or equivalent thereof, an aptamer, and one member of a binding pair, and combinations thereof. Quenching schemes may be used, wherein a quencher and a fluorophore as members of a quenching pair may be used on a probe, such that a change in optical parameters occurs upon binding to the target introduce or quench the signal from the fluorophore. One example of such a system is a molecular beacon. Suitable quencher/fluorophore systems are known in the art. The label may be bound through a variety of intermediate linkages. For example, a polynucleotide may comprise a biotin-binding species, and an optically detectable label may be conjugated to biotin and then bound to the labeled polynucleotide. Similarly, a polynucleotide sensor may comprise an immunological species such as an antibody or fragment, and a secondary antibody containing an optically detectable label may be added.

Chromophores useful in the methods described herein include any substance which can absorb energy and emit light. For multiplexed assays, a plurality of different signaling chromophores can be used with detectably different emission spectra. The chromophore can be a lumophore or a fluorophore. Typical fluorophores include fluorescent dyes, semiconductor nanocrystals, lanthanide chelates, polynucleotide-specific dyes and green fluorescent protein.

Polynucleotides described herein comprise at least 20 consecutive bases of the nucleic acid sequence of a target selected from Table 1 or Table 4 or a complement thereto. The polynucleotides may comprise at least 21, 22, 23, 24, 25, 27, 30, 32, 35, 40, 45, 50, or more consecutive bases of the nucleic acids sequence of a target selected from Table 1 or Table 4, as applicable.

The polynucleotides may be provided in a variety of formats, including as solids, in solution, or in an array. The polynucleotides may optionally comprise one or more labels, which may be chemically and/or enzymatically incorporated into the polynucleotide.

Described herein, one or more polynucleotides provided herein can be provided on a substrate. The substrate can comprise a wide range of material, either biological, nonbiological, organic, inorganic, or a combination of any of these. For example, the substrate may be a polymerized Langmuir Blodgett film, functionalized glass, Si, Ge, GaAs, GaP, SiO₂, SiN₄, modified silicon, or any one of a wide variety of gels or polymers such as (poly)tetrafluoroethylene, (poly)vinylidenedifluoride, polystyrene, cross-linked polystyrene, polyacrylic, polylactic acid, polyglycolic acid, poly(lactide coglycolide), polyanhydrides, poly(methyl methacrylate), poly(ethylene-co-vinyl acetate), polysiloxanes, polymeric silica, latexes, dextran polymers, epoxies, polycarbonates, or combinations thereof. Conducting polymers and photoconductive materials can be used.

The substrate can take the form of an array, a photodiode, an optoelectronic sensor such as an optoelectronic semiconductor chip or optoelectronic thin-film semiconductor, or a biochip. The location(s) of probe(s) on the substrate can be addressable; this can be done in highly dense formats, and the location(s) can be microaddressable or nanoaddressable.

### Diagnostic Samples

A biological sample containing cancer cells is collected from a subject in need of treatment for prostate cancer to evaluate whether a subject will be resistant to ADT. Diagnostic samples for use with the systems and in the methods of the present invention comprise nucleic acids suitable for providing RNAs expression information. In principle, the biological sample from which the expressed RNA is obtained and analyzed for target sequence expression can be any material suspected of comprising prostate cancer tissue or cells. The diagnostic sample can be a biological sample used directly in a method of the invention. Alternatively, the diagnostic sample can be a sample prepared from a biological sample.

In one embodiment, the sample or portion of the sample comprising or suspected of comprising cancer tissue or cells can be any source of biological material, including cells, tissue or fluid, including bodily fluids. Non-limiting examples of the source of the sample include an aspirate, a needle biopsy, a cytology pellet, a bulk tissue preparation or a section thereof obtained for example by surgery or autopsy, lymph fluid, blood, plasma, serum, tumors, and organs. In some embodiments, the sample is from urine. Alternatively, the sample is from blood, plasma or serum. In some embodiments, the sample is from saliva.

The samples may be archival samples, having a known and documented medical outcome, or may be samples from current subjects whose ultimate medical outcome is not yet known.

In some embodiments, the sample may be dissected prior to molecular analysis. The sample may be prepared via macrodissection of a bulk tumor specimen or portion thereof, or may be treated via microdissection, for example via Laser Capture Microdissection (LCM).

The sample may initially be provided in a variety of states, as fresh tissue, fresh frozen tissue, fine needle aspirates, and may be fixed or unfixed. Frequently, medical laboratories routinely prepare medical samples in a fixed state, which facilitates tissue storage. A variety of fixatives can be used to fix tissue to stabilize the morphology of cells, and may be used alone or in combination with other agents. Exemplary fixatives include crosslinking agents, alcohols, acetone, Bouin's solution, Zenker solution, Hely solution, osmic acid solution and Carnoy solution.

Crosslinking fixatives can comprise any agent suitable for forming two or more covalent bonds, for example an aldehyde. Sources of aldehydes typically used for fixation include formaldehyde, paraformaldehyde, glutaraldehyde or formalin. Preferably, the crosslinking agent comprises formaldehyde, which may be included in its native form or in the form of paraformaldehyde or formalin. One of skill in the art would appreciate that for samples in which crosslinking fixatives have been used special preparatory steps may be necessary including for example heating steps and proteinase-k digestion; see methods.

One or more alcohols may be used to fix tissue, alone or in combination with other fixatives. Exemplary alcohols used for fixation include methanol, ethanol and isopropanol.

Formalin fixation is frequently used in medical laboratories. Formalin comprises both an alcohol, typically methanol, and formaldehyde, both of which can act to fix a biological sample.

Whether fixed or unfixed, the biological sample may optionally be embedded in an embedding medium. Exemplary embedding media used in histology including paraffin, Tissue-Tek® V.I.P.TM, Paramat, Paramat Extra, Paraplast, Paraplast X-tra, Paraplast Plus, Peel Away Paraffin Embedding Wax, Polyester Wax, Carbowax Polyethylene Glycol, PolyfinTM, Tissue Freezing Medium TFMFM, Cryo-GefTM, and OCT Compound (Electron Microscopy Sciences, Hatfield, PA). Prior to molecular analysis, the embedding material may be removed via any suitable techniques, as known in the art. For example, where the sample is embedded in wax, the embedding material may be removed by extraction with organic solvent(s), for example xylenes. Kits are commercially available for removing embedding media from tissues. Samples or sections thereof may be subjected to further processing steps as needed, for example serial hydration or dehydration steps.

In some embodiments, the sample is a fixed, wax-embedded biological sample. Frequently, samples from medical laboratories are provided as fixed, wax-embedded samples, most commonly as formalin-fixed, paraffin embedded (FFPE) tissues.

Whatever the source of the biological sample, the target polynucleotide that is ultimately assayed can be prepared synthetically (in the case of control sequences), but typically is purified from the biological source and subjected to one or more preparative steps. The RNA may be purified to remove or diminish one or more undesired components from the biological sample or to concentrate it. Conversely, where the RNA is too concentrated for the particular assay, it may be diluted.

### RNA Extraction

RNA can be extracted and purified from biological samples using any suitable technique. A number of techniques are known in the art, and several are commercially available (e.g., FormaPure nucleic acid extraction kit, Agencourt Biosciences, Beverly MA, High Pure FFPE RNA Micro Kit, Roche Applied Science, Indianapolis, IN). RNA can be extracted from frozen tissue sections using TRIzol (Invitrogen, Carlsbad, CA) and purified using RNeasy Protect kit (Qiagen, Valencia, CA). RNA can be further purified using DNAse I treatment (Ambion, Austin, TX) to eliminate any contaminating DNA. RNA concentrations can be made using a Nanodrop ND-1000 spectrophotometer (Nanodrop Technologies, Rockland, DE). RNA can be further purified to eliminate contaminants that interfere with cDNA synthesis by cold sodium acetate precipitation. RNA integrity can be evaluated by running electropherograms, and RNA integrity number (RIN, a correlative measure that indicates intactness of mRNA) can be determined using the RNA 6000 PicoAssay for the Bioanalyzer 2100 (Agilent Technologies, Santa Clara, CA).

### Kits

Kits for performing the desired method(s) are also provided, and comprise a container or housing for holding the components of the kit, one or more vessels containing one or more nucleic acid(s), and optionally one or more vessels containing one or more reagents. The reagents include those described in the composition of matter section above, and those reagents useful for performing the methods described, including amplification reagents, and may include one or more probes, primers or primer pairs, enzymes (including polymerases and ligases), intercalating dyes, labeled probes, and labels that can be incorporated into amplification products.

Described herein, the kit comprises primers or primer pairs specific for those subsets and combinations of target sequences described herein. The primers or pairs of primers suitable for selectively amplifying the target sequences. The kit may comprise at least two, three, four or five primers or pairs of primers suitable for selectively amplifying one or more targets. The kit may comprise at least 5, 10, 15, 20, 30, 40, 50, 60, 70, 80, 90, 100, 110, or more primers or pairs of primers suitable for selectively amplifying one or more targets.

Described herein, the primers or primer pairs of the kit, when used in an amplification reaction, specifically amplify a non-coding target, coding target, exonic, or non-exonic target described herein, a nucleic acid sequence corresponding to a target selected from Table 1 or Table 4, an RNA form thereof, or a complement to either thereof. The kit may include a plurality of such primers or primer pairs which can specifically amplify a corresponding plurality of different amplify a non-coding target, coding target, exonic, or non-exonic transcript described herein, a nucleic acid sequence corresponding to a target selected from Table 1 or Table 4, RNA forms thereof, or complements thereto. At least two, three, four or five primers or pairs of primers suitable for selectively amplifying the one or more targets can be provided in kit form. Described herein, the kit comprises from five to fifty primers or pairs of primers suitable for amplifying the one or more targets.

The reagents may independently be in liquid or solid form. The reagents may be provided in mixtures. Control samples and/or nucleic acids may optionally be provided in the kit. Control samples may include tissue and/or nucleic acids obtained from or representative of tumor samples from subjects showing no evidence of disease, as well as tissue and/or nucleic acids obtained from or representative of tumor samples from subjects that develop systemic cancer.

The nucleic acids may be provided in an array format, and thus an array or microarray may be included in the kit. The kit optionally may be certified by a government agency for use in prognosing the disease outcome of cancer subjects and/or for designating a treatment modality.

Instructions for using the kit to perform one or more methods of the invention can be provided with the container, and can be provided in any fixed medium. The instructions may be located inside or outside the container or housing, and/or may be printed on the interior or exterior of any surface thereof. A kit may be in multiplex form for concurrently detecting and/or quantitating one or more different target polynucleotides representing the expressed target sequences.

### Amplification and Hybridization

Following sample collection and nucleic acid extraction, the nucleic acid portion of the sample comprising RNA that is or can be used to prepare the target polynucleotide(s) of interest can be subjected to one or more preparative reactions. These preparative reactions can include in vitro transcription (IVT), labeling, fragmentation, amplification and other reactions. mRNA can first be treated with reverse transcriptase and a primer to create cDNA prior to detection, quantitation and/or amplification; this can be done in vitro with purified mRNA or *in situ,* e.g., in cells or tissues affixed to a slide.

By "amplification" is meant any process of producing at least one copy of a nucleic acid, in this case an expressed RNA, and in many cases produces multiple copies. An amplification product can be RNA or DNA, and may include a complementary strand to the expressed target sequence. DNA amplification products can be produced initially through reverse translation and then optionally from further amplification reactions. The amplification product may include all or a portion of a target sequence, and may optionally be labeled. A variety of amplification methods are suitable for use, including polymerase-based methods and ligation-based methods. Exemplary amplification techniques include the polymerase chain reaction method (PCR), the lipase chain reaction (LCR), ribozyme-based methods, self-sustained sequence replication (3SR), nucleic acid sequence-based amplification (NASBA), the use of Q Beta replicase, reverse transcription, nick translation, and the like.

Asymmetric amplification reactions may be used to preferentially amplify one strand representing the target sequence that is used for detection as the target polynucleotide. In some cases, the presence and/or amount of the amplification product itself may be used to determine the expression level of a given target sequence. In other instances, the amplification product may be used to hybridize to an array or other substrate comprising sensor polynucleotides which are used to detect and/or quantitate target sequence expression.

The first cycle of amplification in polymerase-based methods typically forms a primer extension product complementary to the template strand. If the template is single-stranded RNA, a polymerase with reverse transcriptase activity is used in the first amplification to reverse transcribe the RNA to DNA, and additional amplification cycles can be performed to copy the primer extension products. The primers for a PCR must, of course, be designed to hybridize to regions in their corresponding template that can produce an amplifiable segment; thus, each primer must hybridize so that its 3' nucleotide is paired to a nucleotide in its complementary template strand that is located 3' from the 3' nucleotide of the primer used to replicate that complementary template strand in the PCR.

The target polynucleotide can be amplified by contacting one or more strands of the target polynucleotide with a primer and a polymerase having suitable activity to extend the primer and copy the target polynucleotide to produce a full-length complementary polynucleotide or a smaller portion thereof. Any enzyme having a polymerase activity that can copy the target polynucleotide can be used, including DNA polymerases, RNA polymerases, reverse transcriptases, enzymes having more than one type of polymerase or enzyme activity. The enzyme can be thermolabile or thermostable. Mixtures of enzymes can also be used. Exemplary enzymes include: DNA polymerases such as DNA Polymerase I ("Pol I"), the Klenow fragment of Pol I, T4, T7, Sequenase® T7, Sequenase® Version 2.0 T7, *Tub, Taq, Tth, Pfic, Pfu, Tsp, Tfl, Tli* and *Pyrococcus sp* GB-D DNA polymerases; RNA polymerases such as E. *coil,* SP6, T3 and T7 RNA polymerases; and reverse transcriptases such as AMV, M-MuLV, MMLV, RNAse H MMLV (SuperScript®), SuperScript® II, ThermoScript®, HIV-1, and RAV2 reverse transcriptases. All of these enzymes are commercially available. Exemplary polymerases with multiple specificities include RAV2 and *Tli* (exo-) polymerases. Exemplary thermostable polymerases include *Tub, Taq, Tth, Pfic, Pfu, Tsp, Tfl, Tli* and *Pyrococcus sp.* GB-D DNA polymerases.

Suitable reaction conditions are chosen to permit amplification of the target polynucleotide, including pH, buffer, ionic strength, presence and concentration of one or more salts, presence and concentration of reactants and cofactors such as nucleotides and magnesium and/or other metal ions (e.g., manganese), optional cosolvents, temperature, thermal cycling profile for amplification schemes comprising a polymerase chain reaction, and may depend in part on the polymerase being used as well as the nature of the sample. Cosolvents include formamide (typically at from about 2 to about 10 %), glycerol (typically at from about 5 to about 10 %), and DMSO (typically at from about 0.9 to about 10 %). Techniques may be used in the amplification scheme in order to minimize the production of false positives or artifacts produced during amplification. These include "touchdown" PCR, hot-start techniques, use of nested primers, or designing PCR primers so that they form stem-loop structures in the event of primer-dimer formation and thus are not amplified. Techniques to accelerate PCR can be used, for example centrifugal PCR, which allows for greater convection within the sample, and comprising infrared heating steps for rapid heating and cooling of the sample. One or more cycles of amplification can be performed. An excess of one primer can be used to produce an excess of one primer extension product during PCR; preferably, the primer extension product produced in excess is the amplification product to be detected. A plurality of different primers may be used to amplify different target polynucleotides or different regions of a particular target polynucleotide within the sample.

An amplification reaction can be performed under conditions which allow an optionally labeled sensor polynucleotide to hybridize to the amplification product during at least part of an amplification cycle. When the assay is performed in this manner, real-time detection of this hybridization event can take place by monitoring for light emission or fluorescence during amplification, as known in the art.

Where the amplification product is to be used for hybridization to an array or microarray, a number of suitable commercially available amplification products are available. These include amplification kits available from NuGEN, Inc. (San Carlos, CA), including the WT-OvationTm System, WT-OvationTm System v2, WT-OvationTm Pico System, WT-OvationTm FFPE Exon Module, WT-OvationTm FFPE Exon Module RiboAmp and RiboAmp ^{Plus} RNA Amplification Kits (MDS Analytical Technologies (formerly Arcturus) (Mountain View, CA), Genisphere, Inc. (Hatfield, PA), including the RampUp PlusTM and SenseAmpTM RNA Amplification kits, alone or in combination. Amplified nucleic acids may be subjected to one or more purification reactions after amplification and labeling, for example using magnetic beads (e.g., RNAClean magnetic beads, Agencourt Biosciences).

Multiple RNA biomarkers can be analyzed using real-time quantitative multiplex RT-PCR platforms and other multiplexing technologies such as GenomeLab GeXP Genetic Analysis System (Beckman Coulter, Foster City, CA), SmartCycler® 9600 or GeneXpert® Systems (Cepheid, Sunnyvale, CA), ABI 7900 HT Fast Real Time PCR system (Applied Biosystems, Foster City, CA), LightCycler® 480 System (Roche Molecular Systems, Pleasanton, CA), xMAP 100 System (Luminex, Austin, TX) Solexa Genome Analysis System (Illumina, Hayward, CA), OpenArray Real Time qPCR (BioTrove, Woburn, MA) and BeadXpress System (Illumina, Hayward, CA).

### Detection and/or Quantification of Target Sequences

Any method of detecting and/or quantitating the expression of the encoded target sequences can in principle be used in the invention. The expressed target sequences can be directly detected and/or quantitated, or may be copied and/or amplified to allow detection of amplified copies of the expressed target sequences or its complement.

Methods for detecting and/or quantifying a target can include Northern blotting, sequencing, array or microarray hybridization, by enzymatic cleavage of specific structures (e.g., an Invader® assay, Third Wave Technologies, e.g. as described in U.S. Pat. Nos. 5,846,717, 6,090,543; 6,001,567; 5,985,557; and 5,994,069) and amplification methods, e.g. RT-PCR, including in a TaqMan® assay (PE Biosystems, Foster City, Calif., e.g. as described in U.S. Pat. Nos. 5,962,233 and 5,538,848), and may be quantitative or semi-quantitative, and may vary depending on the origin, amount and condition of the available biological sample. Combinations of these methods may also be used. For example, nucleic acids may be amplified, labeled and subjected to microarray analysis.

In some instances, target sequences may be detected by sequencing. Sequencing methods may comprise whole genome sequencing or exome sequencing. Sequencing methods such as Maxim-Gilbert, chain-termination, or high-throughput systems may also be used. Additional, suitable sequencing techniques include classic dideoxy sequencing reactions (Sanger method) using labeled terminators or primers and gel separation in slab or capillary, sequencing by synthesis using reversibly terminated labeled nucleotides, pyrosequencing, 454 sequencing, allele specific hybridization to a library of labeled oligonucleotide probes, sequencing by synthesis using allele specific hybridization to a library of labeled clones that is followed by ligation, real time monitoring of the incorporation of labeled nucleotides during a polymerization step, and SOLiD sequencing.

Additional methods for detecting and/or quantifying a target include single-molecule sequencing (e.g., Helicos, PacBio), sequencing by synthesis (e.g., Illumina, Ion Torrent), sequencing by ligation (e.g., ABI SOLID), sequencing by hybridization (e.g., Complete Genomics), in situ hybridization, bead-array technologies (e.g., Luminex xMAP, Illumina BeadChips), branched DNA technology (e.g., Panomics, Genisphere). Sequencing methods may use fluorescent (e.g., Illumina) or electronic (e.g., Ion Torrent, Oxford Nanopore) methods of detecting nucleotides.

### Reverse Transcription for QRT-PCR Analysis

Reverse transcription can be performed by any method known in the art. For example, reverse transcription may be performed using the Omniscript kit (Qiagen, Valencia, CA), Superscript III kit (Invitrogen, Carlsbad, CA), for RT-PCR. Target-specific priming can be performed in order to increase the sensitivity of detection of target sequences and generate target-specific cDNA.

### TaqMan® Gene Expression Analysis

TaqMan®RT-PCR can be performed using Applied Biosystems Prism (ABI) 7900 HT instruments in a 5 1.11 volume with target sequence-specific cDNA equivalent to 1 ng total RNA.

Primers and probes concentrations for TaqMan analysis are added to amplify fluorescent amplicons using PCR cycling conditions such as 95°C for 10 minutes for one cycle, 95°C for 20 seconds, and 60°C for 45 seconds for 40 cycles. A reference sample can be assayed to ensure reagent and process stability. Negative controls (e.g., no template) should be assayed to monitor any exogenous nucleic acid contamination.

### Classification Arrays

A probe set or probes derived therefrom may be provided in an array format. An "array" is a spatially or logically organized collection of polynucleotide probes. An array comprising probes specific for a coding target, non-coding target, or a combination thereof may be used. Alternatively, an array comprising probes specific for two or more of transcripts of a target selected from Table 1 or Table 4, or a product derived thereof, can be used. Desirably, an array may be specific for 5, 10, 15, 20, 25, 30 or more of transcripts of a target selected from Table 1 or Table 4. Expression of these sequences may be detected alone or in combination with other transcripts. Described herein, an array is used which comprises a wide range of sensor probes for prostate-specific expression products, along with appropriate control sequences. In some instances, the array may comprise the Human Exon 1.0 ST Array (HuEx 1.0 ST, Affymetrix, Inc., Santa Clara, CA.).

Typically the polynucleotide probes are attached to a solid substrate and are ordered so that the location (on the substrate) and the identity of each are known. The polynucleotide probes can be attached to one of a variety of solid substrates capable of withstanding the reagents and conditions necessary for use of the array. Examples include, but are not limited to, polymers, such as (poly)tetrafluoroethylene, (poly)vinylidenedifluoride, polystyrene, polycarbonate, polypropylene and polystyrene; ceramic; silicon; silicon dioxide; modified silicon; (fused) silica, quartz or glass; functionalized glass; paper, such as filter paper; diazotized cellulose; nitrocellulose filter; nylon membrane; and polyacrylamide gel pad. Substrates that are transparent to light are useful for arrays that may be used in an assay that involves optical detection.

Examples of array formats include membrane or filter arrays (for example, nitrocellulose, nylon arrays), plate arrays (for example, multiwell, such as a 24-, 96-, 256-, 384-, 864- or 1536-well, microtitre plate arrays), pin arrays, and bead arrays (for example, in a liquid "slurry"). Arrays on substrates such as glass or ceramic slides are often referred to as chip arrays or "chips." Such arrays are well known in the art. Described herein, the Cancer Prognosticarray is a chip.

### Data Analysis

In some embodiments, one or more pattern recognition methods can be used in analyzing the expression level of target sequences. The pattern recognition method can comprise a linear combination of expression levels, or a nonlinear combination of expression levels. In some embodiments, expression measurements for RNA transcripts or combinations of RNA transcript levels are formulated into linear or non-linear models or algorithms (e.g., an 'expression signature') and converted into a likelihood score. This likelihood score indicates the probability that a biological sample is from a subject who may exhibit no evidence of disease, who may exhibit systemic cancer, or who may exhibit biochemical recurrence. The likelihood score can be used to distinguish these disease states. The models and/or algorithms can be provided in machine readable format, and may be used to correlate expression levels or an expression profile with a disease state, and/or to designate a treatment modality for a subject or class of subjects.

Assaying the expression level for a plurality of targets may comprise the use of an algorithm or classifier. Array data can be managed, classified, and analyzed using techniques known in the art. Assaying the expression level for a plurality of targets may comprise probe set modeling and data pre-processing. Probe set modeling and data pre-processing can be derived using the Robust Multi-Array (RMA) algorithm or variants GC-RMA, *f*RMA. Probe Logarithmic Intensity Error (PLIER) algorithm or variant iterPLIER. Variance or intensity filters can be applied to pre-process data using the RMA algorithm, for example by removing target sequences with a standard deviation of < 10 or a mean intensity of < 100 intensity units of a normalized data range, respectively.

Alternatively, assaying the expression level for a plurality of targets may comprise the use of a machine learning algorithm. The machine learning algorithm may comprise a supervised learning algorithm. Examples of supervised learning algorithms may include Average One-Dependence Estimators (AODE), Artificial neural network (e.g., Backpropagation), Bayesian statistics (e.g., Naive Bayes classifier, Bayesian network, Bayesian knowledge base), Case-based reasoning, Decision trees, Inductive logic programming, Gaussian process regression, Group method of data handling (GMDH), Learning Automata, Learning Vector Quantization, Minimum message length (decision trees, decision graphs, etc.), Lazy learning, Instance-based learning Nearest Neighbor Algorithm, Analogical modeling, Probably approximately correct learning (PAC) learning, Ripple down rules, a knowledge acquisition methodology, Symbolic machine learning algorithms, Subsymbolic machine learning algorithms, Support vector machines, Random Forests, Ensembles of classifiers, Bootstrap aggregating (bagging), and Boosting. Supervised learning may comprise ordinal classification such as regression analysis and Information fuzzy networks (IFN). Alternatively, supervised learning methods may comprise statistical classification, such as AODE, Linear classifiers (e.g., Fisher's linear discriminant, Logistic regression, Naive Bayes classifier, Perceptron, and Support vector machine), quadratic classifiers, k-nearest neighbor, Boosting, Decision trees (e.g., C4.5, Random forests), Bayesian networks, and Hidden Markov models.

The machine learning algorithms may also comprise an unsupervised learning algorithm. Examples of unsupervised learning algorithms may include artificial neural network, Data clustering, Expectation-maximization algorithm, Self-organizing map, Radial basis function network, Vector Quantization, Generative topographic map, Information bottleneck method, and IBSEAD. Unsupervised learning may also comprise association rule learning algorithms such as Apriori algorithm, Eclat algorithm and FP-growth algorithm. Hierarchical clustering, such as Single-linkage clustering and Conceptual clustering, may also be used. Alternatively, unsupervised learning may comprise partitional clustering such as K-means algorithm and Fuzzy clustering.

In some instances, the machine learning algorithms comprise a reinforcement learning algorithm. Examples of reinforcement learning algorithms include, but are not limited to, temporal difference learning, Q-learning and Learning Automata. Alternatively, the machine learning algorithm may comprise Data Pre-processing.

Preferably, the machine learning algorithms may include, but are not limited to, Average One-Dependence Estimators (AODE), Fisher's linear discriminant, Logistic regression, Perceptron, Multilayer Perceptron, Artificial Neural Networks, Support vector machines, Quadratic classifiers, Boosting, Decision trees, C4.5, Bayesian networks, Hidden Markov models, High-Dimensional Discriminant Analysis, and Gaussian Mixture Models. The machine learning algorithm may comprise support vector machines, Naive Bayes classifier, k-nearest neighbor, high-dimensional discriminant analysis, or Gaussian mixture models. In some instances, the machine learning algorithm comprises Random Forests.

### Therapeutic regimens

Diagnosing, predicting, or monitoring a status or outcome of a cancer may comprise treating a cancer or preventing a cancer progression. In addition, diagnosing, predicting, or monitoring a status or outcome of a cancer may comprise identifying or predicting responders or non-responders to an anti-cancer therapy (e.g., ADT). In some instances, diagnosing, predicting, or monitoring may comprise determining a therapeutic regimen. Determining a therapeutic regimen may comprise administering an anti-cancer therapy. Alternatively, determining a therapeutic regimen may comprise modifying, recommending, continuing or discontinuing an anti-cancer regimen. In some instances, if the sample expression patterns are consistent with the expression pattern for a known disease or disease outcome, the expression patterns can be used to designate one or more treatment modalities (e.g., therapeutic regimens, anti-cancer regimen). An anti-cancer regimen may comprise one or more anti-cancer therapies. Examples of anti-cancer therapies include surgery, chemotherapy, radiation therapy, immunotherapy/biological therapy, and photodynamic therapy.

Surgical oncology uses surgical methods to diagnose, stage, and treat cancer, and to relieve certain cancer-related symptoms. Surgery may be used to remove the tumor (e.g., excisions, resections, debulking surgery), reconstruct a part of the body (e.g., restorative surgery), and/or to relieve symptoms such as pain (e.g., palliative surgery). Surgery may also include cryosurgery. Cryosurgery (also called cryotherapy) may use extreme cold produced by liquid nitrogen (or argon gas) to destroy abnormal tissue. Cryosurgery can be used to treat external tumors, such as those on the skin. For external tumors, liquid nitrogen can be applied directly to the cancer cells with a cotton swab or spraying device. Cryosurgery may also be used to treat tumors inside the body (internal tumors and tumors in the bone). For internal tumors, liquid nitrogen or argon gas may be circulated through a hollow instrument called a cryoprobe, which is placed in contact with the tumor. An ultrasound or MRI may be used to guide the cryoprobe and monitor the freezing of the cells, thus limiting damage to nearby healthy tissue. A ball of ice crystals may form around the probe, freezing nearby cells. Sometimes more than one probe is used to deliver the liquid nitrogen to various parts of the tumor. The probes may be put into the tumor during surgery or through the skin (percutaneously). After cryosurgery, the frozen tissue thaws and may be naturally absorbed by the body (for internal tumors), or may dissolve and form a scab (for external tumors).

Chemotherapeutic agents may also be used for the treatment of cancer. Examples of chemotherapeutic agents include alkylating agents, anti-metabolites, plant alkaloids and terpenoids, vinca alkaloids, podophyllotoxin, taxanes, topoisomerase inhibitors, and cytotoxic antibiotics. Cisplatin, carboplatin, and oxaliplatin are examples of alkylating agents. Other alkylating agents include mechlorethamine, cyclophosphamide, chlorambucil, ifosfamide. Alkylating agents may impair cell function by forming covalent bonds with the amino, carboxyl, sulfhydryl, and phosphate groups in biologically important molecules. Alternatively, alkylating agents may chemically modify a cell's DNA.

Anti-metabolites are another example of chemotherapeutic agents. Anti-metabolites may masquerade as purines or pyrimidines and may prevent purines and pyrimidines from becoming incorporated in to DNA during the "S" phase (of the cell cycle), thereby stopping normal development and division. Antimetabolites may also affect RNA synthesis. Examples of metabolites include azathioprine and mercaptopurine.

Alkaloids may be derived from plants and block cell division may also be used for the treatment of cancer. Alkyloids may prevent microtubule function. Examples of alkaloids are vinca alkaloids and taxanes. Vinca alkaloids may bind to specific sites on tubulin and inhibit the assembly of tubulin into microtubules (M phase of the cell cycle). The vinca alkaloids may be derived from the Madagascar periwinkle, *Catharanthus roseus* (formerly known as *Vinca rosea*). Examples of vinca alkaloids include, but are not limited to, vincristine, vinblastine, vinorelbine, or vindesine. Taxanes are diterpenes produced by the plants of the genus *Taxus* (yews). Taxanes may be derived from natural sources or synthesized artificially. Taxanes include paclitaxel (Taxol) and docetaxel (Taxotere). Taxanes may disrupt microtubule function. Microtubules are essential to cell division, and taxanes may stabilize GDP-bound tubulin in the microtubule, thereby inhibiting the process of cell division. Thus, in essence, taxanes may be mitotic inhibitors. Taxanes may also be radiosensitizing and often contain numerous chiral centers.

Alternative chemotherapeutic agents include podophyllotoxin. Podophyllotoxin is a plant-derived compound that may help with digestion and may be used to produce cytostatic drugs such as etoposide and teniposide. They may prevent the cell from entering the G1 phase (the start of DNA replication) and the replication of DNA (the S phase).

Topoisomerases are essential enzymes that maintain the topology of DNA. Inhibition of type I or type II topoisomerases may interfere with both transcription and replication of DNA by upsetting proper DNA supercoiling. Some chemotherapeutic agents may inhibit topoisomerases. For example, some type I topoisomerase inhibitors include *camptothecins*: irinotecan and topotecan. Examples of type II inhibitors include amsacrine, etoposide, etoposide phosphate, and teniposide.

Another example of chemotherapeutic agents is cytotoxic antibiotics. Cytotoxic antibiotics are a group of antibiotics that are used for the treatment of cancer because they may interfere with DNA replication and/or protein synthesis. Cytotoxic antiobiotics include, but are not limited to, actinomycin, anthracyclines, doxorubicin, daunorubicin, valrubicin, idarubicin, epirubicin, bleomycin, plicamycin, and mitomycin.

In some instances, the anti-cancer treatment may comprise radiation therapy. Radiation can come from a machine outside the body (external-beam radiation therapy) or from radioactive material placed in the body near cancer cells (internal radiation therapy, more commonly called brachytherapy). Systemic radiation therapy uses a radioactive substance, given by mouth or into a vein that travels in the blood to tissues throughout the body.

External-beam radiation therapy may be delivered in the form of photon beams (either x-rays or gamma rays). A photon is the basic unit of light and other forms of electromagnetic radiation. An example of external-beam radiation therapy is called 3-dimensional conformal radiation therapy (3D-CRT). 3D-CRT may use computer software and advanced treatment machines to deliver radiation to very precisely shaped target areas. Many other methods of external-beam radiation therapy are currently being tested and used in cancer treatment. These methods include, but are not limited to, intensity-modulated radiation therapy (IMRT), image-guided radiation therapy (IGRT), Stereotactic radiosurgery (SRS), Stereotactic body radiation therapy (SBRT), and proton therapy.

Intensity-modulated radiation therapy (IMRT) is an example of external-beam radiation and may use hundreds of tiny radiation beam-shaping devices, called collimators, to deliver a single dose of radiation. The collimators can be stationary or can move during treatment, allowing the intensity of the radiation beams to change during treatment sessions. This kind of dose modulation allows different areas of a tumor or nearby tissues to receive different doses of radiation. IMRT is planned in reverse (called inverse treatment planning). In inverse treatment planning, the radiation doses to different areas of the tumor and surrounding tissue are planned in advance, and then a high-powered computer program calculates the required number of beams and angles of the radiation treatment. In contrast, during traditional (forward) treatment planning, the number and angles of the radiation beams are chosen in advance and computers calculate how much dose may be delivered from each of the planned beams. The goal of IMRT is to increase the radiation dose to the areas that need it and reduce radiation exposure to specific sensitive areas of surrounding normal tissue.

Another example of external-beam radiation is image-guided radiation therapy (IGRT). In IGRT, repeated imaging scans (CT, MRI, or PET) may be performed during treatment. These imaging scans may be processed by computers to identify changes in a tumor's size and location due to treatment and to allow the position of the subject or the planned radiation dose to be adjusted during treatment as needed. Repeated imaging can increase the accuracy of radiation treatment and may allow reductions in the planned volume of tissue to be treated, thereby decreasing the total radiation dose to normal tissue.

Tomotherapy is a type of image-guided IMRT. A tomotherapy machine is a hybrid between a CT imaging scanner and an external-beam radiation therapy machine. The part of the tomotherapy machine that delivers radiation for both imaging and treatment can rotate completely around the subject in the same manner as a normal CT scanner. Tomotherapy machines can capture CT images of the subject's tumor immediately before treatment sessions, to allow for very precise tumor targeting and sparing of normal tissue.

Stereotactic radiosurgery (SRS) can deliver one or more high doses of radiation to a small tumor. SRS uses extremely accurate image-guided tumor targeting and subject positioning. Therefore, a high dose of radiation can be given without excess damage to normal tissue. SRS can be used to treat small tumors with well-defined edges. It is most commonly used in the treatment of brain or spinal tumors and brain metastases from other cancer types. For the treatment of some brain metastases, subjects may receive radiation therapy to the entire brain (called whole-brain radiation therapy) in addition to SRS. SRS requires the use of a head frame or other device to immobilize the subject during treatment to ensure that the high dose of radiation is delivered accurately.

Stereotactic body radiation therapy (SBRT) delivers radiation therapy in fewer sessions, using smaller radiation fields and higher doses than 3D-CRT in most cases. SBRT may treat tumors that lie outside the brain and spinal cord. Because these tumors are more likely to move with the normal motion of the body, and therefore cannot be targeted as accurately as tumors within the brain or spine, SBRT is usually given in more than one dose. SBRT can be used to treat small, isolated tumors, including cancers in the lung and liver. SBRT systems may be known by their brand names, such as the CyberKnife®.

In proton therapy, external-beam radiation therapy may be delivered by proton. Protons are a type of charged particle. Proton beams differ from photon beams mainly in the way they deposit energy in living tissue. Whereas photons deposit energy in small packets all along their path through tissue, protons deposit much of their energy at the end of their path (called the Bragg peak) and deposit less energy along the way. Use of protons may reduce the exposure of normal tissue to radiation, possibly allowing the delivery of higher doses of radiation to a tumor.

Other charged particle beams such as electron beams may be used to irradiate superficial tumors, such as skin cancer or tumors near the surface of the body, but they cannot travel very far through tissue.

Internal radiation therapy (brachytherapy) is radiation delivered from radiation sources (radioactive materials) placed inside or on the body. Several brachytherapy techniques are used in cancer treatment. Interstitial brachytherapy may use a radiation source placed within tumor tissue, such as within a prostate tumor. Intracavitary brachytherapy may use a source placed within a surgical cavity or a body cavity, such as the chest cavity, near a tumor. Episcleral brachytherapy, which may be used to treat melanoma inside the eye, may use a source that is attached to the eye. In brachytherapy, radioactive isotopes can be sealed in tiny pellets or "seeds." These seeds may be placed in subjects using delivery devices, such as needles, catheters, or some other type of carrier. As the isotopes decay naturally, they give off radiation that may damage nearby cancer cells. Brachytherapy may be able to deliver higher doses of radiation to some cancers than external-beam radiation therapy while causing less damage to normal tissue.

Brachytherapy can be given as a low-dose-rate or a high-dose-rate treatment. In low-dose-rate treatment, cancer cells receive continuous low-dose radiation from the source over a period of several days. In high-dose-rate treatment, a robotic machine attached to delivery tubes placed inside the body may guide one or more radioactive sources into or near a tumor, and then removes the sources at the end of each treatment session. High-dose-rate treatment can be given in one or more treatment sessions. An example of a high-dose-rate treatment is the MammoSite® system. Bracytherapy may be used to treat subjects with breast cancer who have undergone breast-conserving surgery.

The placement of brachytherapy sources can be temporary or permanent. For permanent brachytherapy, the sources may be surgically sealed within the body and left there, even after all of the radiation has been given off. In some instances, the remaining material (in which the radioactive isotopes were sealed) does not cause any discomfort or harm to the subject. Permanent brachytherapy is a type of low-dose-rate brachytherapy. For temporary brachytherapy, tubes (catheters) or other carriers are used to deliver the radiation sources, and both the carriers and the radiation sources are removed after treatment. Temporary brachytherapy can be either low-dose-rate or high-dose-rate treatment. Brachytherapy may be used alone or in addition to external-beam radiation therapy to provide a "boost" of radiation to a tumor while sparing surrounding normal tissue.

In systemic radiation therapy, a subject may swallow or receive an injection of a radioactive substance, such as radioactive iodine or a radioactive substance bound to a monoclonal antibody. Radioactive iodine (1311) is a type of systemic radiation therapy commonly used to help treat cancer, such as thyroid cancer. Thyroid cells naturally take up radioactive iodine. For systemic radiation therapy for some other types of cancer, a monoclonal antibody may help target the radioactive substance to the right place. The antibody joined to the radioactive substance travels through the blood, locating and killing tumor cells. For example, the drug ibritumomab tiuxetan (Zevalin®) may be used for the treatment of certain types of B-cell non-Hodgkin lymphoma (NHL). The antibody part of this drug recognizes and binds to a protein found on the surface of B lymphocytes. The combination drug regimen of tositumomab and iodine I 131 tositumomab (Bexxar®) may be used for the treatment of certain types of cancer, such as NHL. In this regimen, nonradioactive tositumomab antibodies may be given to subjects first, followed by treatment with tositumomab antibodies that have 1311 attached. Tositumomab may recognize and bind to the same protein on B lymphocytes as ibritumomab. The nonradioactive form of the antibody may help protect normal B lymphocytes from being damaged by radiation from 131I.

Some systemic radiation therapy drugs relieve pain from cancer that has spread to the bone (bone metastases). This is a type of palliative radiation therapy. The radioactive drugs samarium-153-lexidronam (Quadramet®) and strontium-89 chloride (Metastron®) are examples of radiopharmaceuticals may be used to treat pain from bone metastases.

Biological therapy (sometimes called immunotherapy, biotherapy, biologic therapy, or biological response modifier (BRM) therapy) uses the body's immune system, either directly or indirectly, to fight cancer or to lessen the side effects that may be caused by some cancer treatments. Biological therapies include interferons, interleukins, colony-stimulating factors, monoclonal antibodies, vaccines, gene therapy, and nonspecific immunomodulating agents.

Interferons (IFNs) are types of cytokines that occur naturally in the body. Interferon alpha, interferon beta, and interferon gamma are examples of interferons that may be used in cancer treatment.

Like interferons, interleukins (ILs) are cytokines that occur naturally in the body and can be made in the laboratory. Many interleukins have been identified for the treatment of cancer. For example, interleukin-2 (IL-2 or aldesleukin), interleukin 7, and interleukin 12 have may be used as an anti-cancer treatment. IL-2 may stimulate the growth and activity of many immune cells, such as lymphocytes, that can destroy cancer cells. Interleukins may be used to treat a number of cancers, including leukemia, lymphoma, and brain, colorectal, ovarian, breast, kidney and prostate cancers.

Colony-stimulating factors (CSFs) (sometimes called hematopoietic growth factors) may also be used for the treatment of cancer. Some examples of CSFs include, but are not limited to, G-CSF (filgrastim) and GM-CSF (sargramostim). CSFs may promote the division of bone marrow stem cells and their development into white blood cells, platelets, and red blood cells. Bone marrow is critical to the body's immune system because it is the source of all blood cells. Because anticancer drugs can damage the body's ability to make white blood cells, red blood cells, and platelets, stimulation of the immune system by CSFs may benefit subjects undergoing other anti-cancer treatment, thus CSFs may be combined with other anti-cancer therapies, such as chemotherapy. CSFs may be used to treat a large variety of cancers, including lymphoma, leukemia, multiple myeloma, melanoma, and cancers of the brain, lung, esophagus, breast, uterus, ovary, prostate, kidney, colon, and rectum.

Another type of biological therapy includes monoclonal antibodies (MOABs or MoABs). These antibodies may be produced by a single type of cell and may be specific for a particular antigen. To create MOABs, a human cancer cells may be injected into mice. In response, the mouse immune system can make antibodies against these cancer cells. The mouse plasma cells that produce antibodies may be isolated and fused with laboratory-grown cells to create "hybrid" cells called hybridomas. Hybridomas can indefinitely produce large quantities of these pure antibodies, or MOABs. MOABs may be used in cancer treatment in a number of ways. For instance, MOABs that react with specific types of cancer may enhance a subject's immune response to the cancer. MOABs can be programmed to act against cell growth factors, thus interfering with the growth of cancer cells.

MOABs may be linked to other anti-cancer therapies such as chemotherapeutics, radioisotopes (radioactive substances), other biological therapies, or other toxins. When the antibodies latch onto cancer cells, they deliver these anti-cancer therapies directly to the tumor, helping to destroy it. MOABs carrying radioisotopes may also prove useful in diagnosing certain cancers, such as colorectal, ovarian, and prostate.

Rituxan® (rituximab) and Herceptin® (trastuzumab) are examples of MOABs that may be used as a biological therapy. Rituxan may be used for the treatment of non-Hodgkin lymphoma. Herceptin can be used to treat metastatic breast cancer in subjects with tumors that produce excess amounts of a protein called HER2. Alternatively, MOABs may be used to treat lymphoma, leukemia, melanoma, and cancers of the brain, breast, lung, kidney, colon, rectum, ovary, prostate, and other areas.

Cancer vaccines are another form of biological therapy. Cancer vaccines may be designed to encourage the subject's immune system to recognize cancer cells. Cancer vaccines may be designed to treat existing cancers (therapeutic vaccines) or to prevent the development of cancer (prophylactic vaccines). Therapeutic vaccines may be injected in a person after cancer is diagnosed. These vaccines may stop the growth of existing tumors, prevent cancer from recurring, or eliminate cancer cells not killed by prior treatments. Cancer vaccines given when the tumor is small may be able to eradicate the cancer. On the other hand, prophylactic vaccines are given to healthy individuals before cancer develops. These vaccines are designed to stimulate the immune system to attack viruses that can cause cancer. By targeting these cancer-causing viruses, development of certain cancers may be prevented. For example, cervarix and gardasil are vaccines to treat human papilloma virus and may prevent cervical cancer. Therapeutic vaccines may be used to treat melanoma, lymphoma, leukemia, and cancers of the brain, breast, lung, kidney, ovary, prostate, pancreas, colon, and rectum. Cancer vaccines can be used in combination with other anti-cancer therapies.

Gene therapy is another example of a biological therapy. Gene therapy may involve introducing genetic material into a person's cells to fight disease. Gene therapy methods may improve a subject's immune response to cancer. For example, a gene may be inserted into an immune cell to enhance its ability to recognize and attack cancer cells. In another approach, cancer cells may be injected with genes that cause the cancer cells to produce cytokines and stimulate the immune system.

In some instances, biological therapy includes nonspecific immunomodulating agents. Nonspecific immunomodulating agents are substances that stimulate or indirectly augment the immune system. Often, these agents target key immune system cells and may cause secondary responses such as increased production of cytokines and immunoglobulins. Two nonspecific immunomodulating agents used in cancer treatment are bacillus Calmette-Guerin (BCG) and levamisole. BCG may be used in the treatment of superficial bladder cancer following surgery. BCG may work by stimulating an inflammatory, and possibly an immune, response. A solution of BCG may be instilled in the bladder. Levamisole is sometimes used along with fluorouracil (5-FU) chemotherapy in the treatment of stage III (Dukes' C) colon cancer following surgery. Levamisole may act to restore depressed immune function.

Photodynamic therapy (PDT) is an anti-cancer treatment that may use a drug, called a photosensitizer or photosensitizing agent, and a particular type of light. When photosensitizers are exposed to a specific wavelength of light, they may produce a form of oxygen that kills nearby cells. A photosensitizer may be activated by light of a specific wavelength. This wavelength determines how far the light can travel into the body. Thus, photosensitizers and wavelengths of light may be used to treat different areas of the body with PDT.

In the first step of PDT for cancer treatment, a photosensitizing agent may be injected into the bloodstream. The agent may be absorbed by cells all over the body but may stay in cancer cells longer than it does in normal cells. Approximately 24 to 72 hours after injection, when most of the agent has left normal cells but remains in cancer cells, the tumor can be exposed to light. The photosensitizer in the tumor can absorb the light and produces an active form of oxygen that destroys nearby cancer cells. In addition to directly killing cancer cells, PDT may shrink or destroy tumors in two other ways. The photosensitizer can damage blood vessels in the tumor, thereby preventing the cancer from receiving necessary nutrients. PDT may also activate the immune system to attack the tumor cells.

The light used for PDT can come from a laser or other sources. Laser light can be directed through fiber optic cables (thin fibers that transmit light) to deliver light to areas inside the body. For example, a fiber optic cable can be inserted through an endoscope (a thin, lighted tube used to look at tissues inside the body) into the lungs or esophagus to treat cancer in these organs. Other light sources include light-emitting diodes (LEDs), which may be used for surface tumors, such as skin cancer. PDT is usually performed as an outsubject procedure. PDT may also be repeated and may be used with other therapies, such as surgery, radiation, or chemotherapy.

Extracorporeal photopheresis (ECP) is a type of PDT in which a machine may be used to collect the subject's blood cells. The subject's blood cells may be treated outside the body with a photosensitizing agent, exposed to light, and then returned to the subject. ECP may be used to help lessen the severity of skin symptoms of cutaneous T-cell lymphoma that has not responded to other therapies. ECP may be used to treat other blood cancers, and may also help reduce rejection after transplants.

Additionally, photosensitizing agent, such as porfimer sodium or Photofrin®, may be used in PDT to treat or relieve the symptoms of esophageal cancer and non-small cell lung cancer. Porfimer sodium may relieve symptoms of esophageal cancer when the cancer obstructs the esophagus or when the cancer cannot be satisfactorily treated with laser therapy alone. Porfimer sodium may be used to treat non-small cell lung cancer in subjects for whom the usual treatments are not appropriate, and to relieve symptoms in subjects with non-small cell lung cancer that obstructs the airways. Porfimer sodium may also be used for the treatment of precancerous lesions in subjects with Barrett esophagus, a condition that can lead to esophageal cancer.

Laser therapy may use high-intensity light to treat cancer and other illnesses. Lasers can be used to shrink or destroy tumors or precancerous growths. Lasers are most commonly used to treat superficial cancers (cancers on the surface of the body or the lining of internal organs) such as basal cell skin cancer and the very early stages of some cancers, such as cervical, penile, vaginal, vulvar, and non-small cell lung cancer.

Lasers may also be used to relieve certain symptoms of cancer, such as bleeding or obstruction. For example, lasers can be used to shrink or destroy a tumor that is blocking a subject's trachea (windpipe) or esophagus. Lasers also can be used to remove colon polyps or tumors that are blocking the colon or stomach.

Laser therapy is often given through a flexible endoscope (a thin, lighted tube used to look at tissues inside the body). The endoscope is fitted with optical fibers (thin fibers that transmit light). It is inserted through an opening in the body, such as the mouth, nose, anus, or vagina. Laser light is then precisely aimed to cut or destroy a tumor.

Laser-induced interstitial thermotherapy (LITT), or interstitial laser photocoagulation, also uses lasers to treat some cancers. LITT is similar to a cancer treatment called hyperthermia, which uses heat to shrink tumors by damaging or killing cancer cells. During LITT, an optical fiber is inserted into a tumor. Laser light at the tip of the fiber raises the temperature of the tumor cells and damages or destroys them. LITT is sometimes used to shrink tumors in the liver.

Laser therapy can be used alone, but most often it is combined with other treatments, such as surgery, chemotherapy, or radiation therapy. In addition, lasers can seal nerve endings to reduce pain after surgery and seal lymph vessels to reduce swelling and limit the spread of tumor cells.

Lasers used to treat cancer may include carbon dioxide (CO₂) lasers, argon lasers, and neodymium:yttrium-aluminum-garnet (Nd:YAG) lasers. Each of these can shrink or destroy tumors and can be used with endoscopes. CO₂ and argon lasers can cut the skin's surface without going into deeper layers. Thus, they can be used to remove superficial cancers, such as skin cancer. In contrast, the Nd:YAG laser is more commonly applied through an endoscope to treat internal organs, such as the uterus, esophagus, and colon. Nd:YAG laser light can also travel through optical fibers into specific areas of the body during LITT. Argon lasers are often used to activate the drugs used in PDT.

For subjects with high test scores consistent with systemic disease outcome after prostatectomy, additional treatment modalities such as adjuvant chemotherapy (e.g., docetaxel, mitoxantrone and prednisone), systemic radiation therapy (e.g., samarium or strontium) and/or anti-androgen therapy (e.g., surgical castration, finasteride, dutasteride) can be designated. Such subjects would likely be treated immediately with anti-androgen therapy alone or in combination with radiation therapy in order to eliminate presumed micro-metastatic disease, which cannot be detected clinically but can be revealed by the target sequence expression signature.

Such subjects can also be more closely monitored for signs of disease progression. For subjects with intermediate test scores consistent with biochemical recurrence only (BCR-only or elevated PSA that does not rapidly become manifested as systemic disease only localized adjuvant therapy (e.g., radiation therapy of the prostate bed) or short course of anti-androgen therapy would likely be administered. For subjects with low scores or scores consistent with no evidence of disease (NED) adjuvant therapy would not likely be recommended by their physicians in order to avoid treatment-related side effects such as metabolic syndrome (e.g., hypertension, diabetes and/or weight gain), osteoporosis, proctitis, incontinence or impotence. Subjects with samples consistent with NED could be designated for watchful waiting, or for no treatment. Subjects with test scores that do not correlate with systemic disease but who have successive PSA increases could be designated for watchful waiting, increased monitoring, or lower dose or shorter duration anti-androgen therapy.

Target sequences can be grouped so that information obtained about the set of target sequences in the group can be used to make or assist in making a clinically relevant judgment such as a diagnosis, prognosis, or treatment choice.

A subject report is also provided comprising a representation of measured expression levels of a plurality of target sequences in a biological sample from the subject, wherein the representation comprises expression levels of target sequences corresponding to any one, two, three, four, five, six, eight, ten, twenty, thirty or more of the target sequences corresponding to a target selected from Table 1 or Table 4, the subsets described herein, or a combination thereof. In some embodiments, the representation of the measured expression level(s) may take the form of a linear or nonlinear combination of expression levels of the target sequences of interest. The subject report may be provided in a machine (e.g., a computer) readable format and/or in a hard (paper) copy. The report can also include standard measurements of expression levels of said plurality of target sequences from one or more sets of subjects with known disease status and/or outcome. The report can be used to inform the subject and/or treating physician of the expression levels of the expressed target sequences, the likely medical diagnosis and/or implications, and optionally may recommend a treatment modality for the subject.

Also provided are representations of the gene expression profiles useful for treating, diagnosing, prognosticating, and otherwise assessing disease. In some embodiments, these profile representations are reduced to a medium that can be automatically read by a machine such as computer readable media (magnetic, optical, and the like). The articles can also include instructions for assessing the gene expression profiles in such media. For example, the articles may comprise a readable storage form having computer instructions for comparing gene expression profiles of the portfolios of genes described above. The articles may also have gene expression profiles digitally recorded therein so that they may be compared with gene expression data from subject samples. Alternatively, the profiles can be recorded in different representational format. A graphical recordation is one such format. Clustering algorithms can assist in the visualization of such data.

### Treatment Response Prediction

Higher ARS scores correlate with development of resistance to ADT. In some embodiments, the methods of the present invention are useful for predicting development of resistance to ADT following radical prostatectomy or in metastatic castration resistant prostate cancer.

In other embodiments, the methods of the present invention are useful for predicting survival outcomes of subjects. Higher ARS scores correlate with shorter periods of metastasis-free survival.

Subjects with higher ARS scores tend to fail treatment with ADT and are better candidates for other treatment options.

### Diagnostic System and Computerized Methods for Diagnosis of Prostate Cancer

Described herein is a computer implemented method for predicting resistance to androgen deprivation therapy (ADT) for a subject who has prostate cancer. The computer performs steps comprising: receiving inputted subject data comprising values for the levels of one or more prostate cancer biomarkers in a biological sample from the subject; analyzing the levels of one or more biomarkers and comparing with respective reference value ranges for the biomarkers; calculating an ARS score for the subject; calculating the likelihood that the subject is resistant to ADT; and displaying information regarding the diagnosis of the subject. In certain embodiments, the inputted subject data comprises values for the levels of expression of a plurality of prostate cancer biomarkers in a biological sample from the subject. Described herein, the inputted subject data comprises values for the levels of expression of biomarker genes comprising SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12, NLRP13, MPP7, CFL1, DESI2, OR51E2, KCNMB1, DLGAP1, SPRR1A, CROT, KIFC1, POLD4, CASP2, WHSC1, MPZL2, NAV1, RNF168, FOXM1, ZC3H11A, FAM3D, KCNK17, PLXNA2, SUOX, ANP32E, REST, NKX2.2, RBBP8, NSMCE4A, H19, ATP1A2, PLXNC1, NUP62, ACAA2, ADH1C, THYN1, COX7A2L, MAP1B or a combination thereof. In another embodiment, the inputted subject data further comprises values for the levels of expression of one or more biomarker genes selected from SELE, B3GALTL, GABRB3, CLEC9A, PRKAG1, SLC35F2, CRYM, FBXO43, GALM, TMEM133, TFAP4, KCNH8, KIAA1210, LRRC18, PEX11A, CCDC151, MORN3, GLYATL1, EHHADH, LPGAT1, FAM134A, RLN1, DNAJC12, TXK, TM2D2, CDH26, RBPMS2, NUDT1, STMN1, EZH2, NAV2, SEMA3C, CCL16, HJURP, SOX14, NPR3, HEY2, SFTA3, C8orf4, PRTFDC1, HEPN1, ID2, ALDH2, LSM7, FAHD2A, TACC2, MSMB, KLK12, NLRP13 or a combination thereof.

Described herein is a computer implemented method for predicting resistance to androgen deprivation therapy (ADT) for a subject who has prostate cancer. The computer performs steps comprising: receiving inputted subject data comprising values for the levels of one or more prostate cancer biomarkers in a biological sample from the subject; analyzing the levels of one or more biomarkers and comparing with respective reference value ranges for the biomarkers; calculating an ARS score for the subject; calculating the likelihood that the subject is resistant to ADT; and displaying information regarding the diagnosis of the subject. In certain embodiments, the inputted subject data comprises values for the levels of expression of a plurality of prostate cancer biomarkers in a biological sample from the subject. Described herein, the inputted subject data comprises values for the levels of expression of biomarker genes comprising DAND5, GABRB3, RIMS2, SNCAIP, TMEM176A, KCNMB2, PLEKHH2, AGTR1, BAG3, C10orf81, C8orf4, CRISP2, GALM, GHR, RRAS, SPATA13, TMEFF2, ARHGAP11A, GNAZ, AC084018.1, RRM2, TFAP4, HEPN1, MPDU1, RLN1, SELE, WDR93, ATF5, HEY2, CREB3L1, SLC35F2, FAM134A, NSMCE4A or a combination thereof. Described herein, the inputted subject data further comprises values for the levels of expression of one or more biomarker genes selected from KLK12, NAV2, POGK, TET1, ELL2, ADAMTS14, CDKN2C, MUC1, TNIK, POLD4, ASB16, CASP2, FAM57B, FOXM1, NAV1, KIF1C, NUP210, CDH3, and TRPV6. Described herein, the inputted subject data comprises values for the levels of expression of biomarker genes comprising DAND5, GABRB3, KLK12, NAV2, POGK, RIMS2, SNCAIP, TET1, TMEM176A, ELL2, ADAMTS14, CDKN2C, KCNMB2, MUC1, PLEKHH2, TNIK, AGTR1, BAG3, C10orf81, C8orf4, CRISP2, GALM, GHR, POLD4, RRAS, SPATA13, TMEFF2, ARHGAP11A, ASB16, CASP2, FAM57B, FOXM1, GNAZ, AC084018.1, NAV1, RRM2, TFAP4, HEPN1, KIF1C, MPDU1, RLN1, SELE, WDR93, ATF5, HEY2, NUP210, CDH3, CREB3L1, SLC35F2, TRPV6, FAM134A, NSMCE4A or a combination thereof.

Described herein is a diagnostic system for performing the computer implemented method, as described. A diagnostic system may include a computer containing a processor, a storage component (i.e., memory), a display component, and other components typically present in general purpose computers. The storage component stores information accessible by the processor, including instructions that may be executed by the processor and data that may be retrieved, manipulated or stored by the processor.

The storage component includes instructions for determining the diagnosis of the subject. For example, the storage component includes instructions for calculating a ARS score for the subject based on biomarker expression levels, as described herein (see Examples). The computer processor is coupled to the storage component and configured to execute the instructions stored in the storage component in order to receive subject data and analyze subject data according to one or more algorithms. The display component displays information regarding the diagnosis of the subject.

The storage component may be of any type capable of storing information accessible by the processor, such as a hard-drive, memory card, ROM, RAM, DVD, CD-ROM, USB Flash drive, write-capable, and read-only memories. The processor may be any well-known processor, such as processors from Intel Corporation. Alternatively, the processor may be a dedicated controller such as an ASIC.

The instructions may be any set of instructions to be executed directly (such as machine code) or indirectly (such as scripts) by the processor. In that regard, the terms "instructions," "steps" and "programs" may be used interchangeably herein. The instructions may be stored in object code form for direct processing by the processor, or in any other computer language including scripts or collections of independent source code modules that are interpreted on demand or compiled in advance.

Data may be retrieved, stored or modified by the processor in accordance with the instructions. For instance, although the diagnostic system is not limited by any particular data structure, the data may be stored in computer registers, in a relational database as a table having a plurality of different fields and records, XML documents, or flat files. The data may also be formatted in any computer-readable format such as, but not limited to, binary values, ASCII or Unicode. Moreover, the data may comprise any information sufficient to identify the relevant information, such as numbers, descriptive text, proprietary codes, pointers, references to data stored in other memories (including other network locations) or information which is used by a function to calculate the relevant data.

Described herein, the processor and storage component may comprise multiple processors and storage components that may or may not be stored within the same physical housing. For example, some of the instructions and data may be stored on a removable DVD, and others within a read-only computer chip. Some or all of the instructions and data may be stored in a location physically remote from, yet still accessible by, the processor. Similarly, the processor may actually comprise a collection of processors which may or may not operate in parallel.

Described herein, the computer is a server communicating with one or more client computers. Each client computer may be configured similarly to the server, with a processor, storage component and instructions. Each client computer may be a personal computer, intended for use by a person, having all the internal components normally found in a personal computer such as a central processing unit (CPU), display (for example, a monitor displaying information processed by the processor), DVD, hard-drive, user input device (for example, a mouse, keyboard, touch-screen or microphone), speakers, modem and/or network interface device (telephone, cable or otherwise) and all of the components used for connecting these elements to one another and permitting them to communicate (directly or indirectly) with one another. Moreover, computers in accordance with the systems and methods described herein may comprise any device capable of processing instructions and transmitting data to and from humans and other computers including network computers lacking local storage capability.

Although the client computers may comprise a full-sized personal computer, many aspects of the system and method are particularly advantageous when used in connection with mobile devices capable of wirelessly exchanging data with a server over a network such as the Internet. For example, client computer may be a wireless-enabled PDA such as a Blackberry phone, Apple iPhone, Android phone, or other Internet-capable cellular phone. In such regard, the user may input information using a small keyboard, a keypad, a touch screen, or any other means of user input. The computer may have an antenna for receiving a wireless signal.

The server and client computers are capable of direct and indirect communication, such as over a network. It should be appreciated that a typical system can include a large number of connected computers, with each different computer being at a different node of the network. The network, and intervening nodes, may comprise various combinations of devices and communication protocols including the Internet, World Wide Web, intranets, virtual private networks, wide area networks, local networks, cell phone networks, private networks using communication protocols proprietary to one or more companies, Ethernet, WiFi and HTTP. Such communication may be facilitated by any device capable of transmitting data to and from other computers, such as modems (e.g., dial-up or cable), networks and wireless interfaces. The server may be a web server.

Although certain advantages are obtained when information is transmitted or received as noted above, other aspects of the system and method are not limited to any particular manner of transmission of information. For example, in some aspects, information may be sent via a medium such as a disk, tape, flash drive, memory card, DVD, or CD-ROM. In other aspects, the information may be transmitted in a non-electronic format and manually entered into the system. Yet further, although some functions are indicated as taking place on a server and others on a client, various aspects of the system and method may be implemented by a single computer having a single processor.

### EXAMPLES

Below are examples of specific embodiments for carrying out the present invention. The examples are offered for illustrative purposes only, and are not intended to limit the scope of the present invention in any way.

Efforts have been made to ensure accuracy with respect to numbers used (e.g., amounts, temperatures, etc.), but some experimental error and deviation should, of course, be allowed for.

### Example 1: Development and Assessment of a Genomic Classifier to Predict Hormone Treatment Failure.

A genomic classifier to predict hormone treatment failure in prostate cancer subjects was developed as follows. Neuroendocrine prostate cancer (NEPC) can be less sensitive or even resistant to androgen deprivation therapy (ADT), which is one of the main treatment options for locally advanced, recurrent and metastatic prostate cancer. We collected a set of 1,557 genes to build a genomic classifier to predict response to ADT.

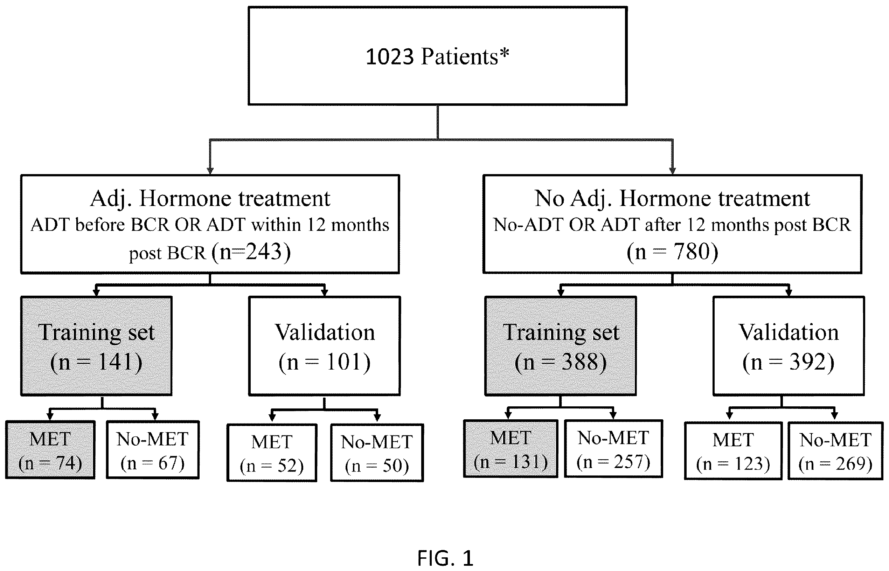

A total of 529 subject prostate cancer expression profiles from formalin-fixed, paraffin-embedded (FFPE) tissues were analyzed from the Decipher GRID database and used as a training set to develop an androgen deprivation therapy (ADT) resistance signature (ARS). Training data was collected from a nested case-control design cohort from the Mayo Clinic tumor registry, which was used previously to develop the Decipher prostate cancer classifier (Erho et al. (2013) PLoS One 8(6):e66855). In the training cohort, 205 subjects experienced metastasis. Subjects with hormonal treatment within 12 months of radical prostatectomy (RP) defined the adjuvant ADT arm, and subjects with treatment after 12 months or no treatment defined the no treatment arm. As a result 141 subjects were in the ADT treated arm of which 74 subjects developed metastasis (failed ADT), and 388 subjects were in the no treated arm of which 131 failed ADT (FIG. 1).

A representative FFPE block from the index prostate cancer lesion was identified by pathologists. RNA was extracted from tumor tissues from the representative FFPE blocks, amplified using the Ovation WTA FFPE system (NuGen, San Carlos, CA, USA), and labeled and hybridized to Human Exon 1.0 ST microarrays (Affymetrix, Santa Clara, CA) covering 1.4 million probesets that were summarized to ∼22,000 core-level gene expression profiles. Analysis and generation of signature scores were performed as previously described in (Erho et al., *supra*) and quality control was verified using Affymetrix power tools (Lockstone et al. (2011) Bioinform. 12(6):634-644). All data normalization used the SCAN algorithm (Piccolo et al. (2012) Genomics 100:337-344).

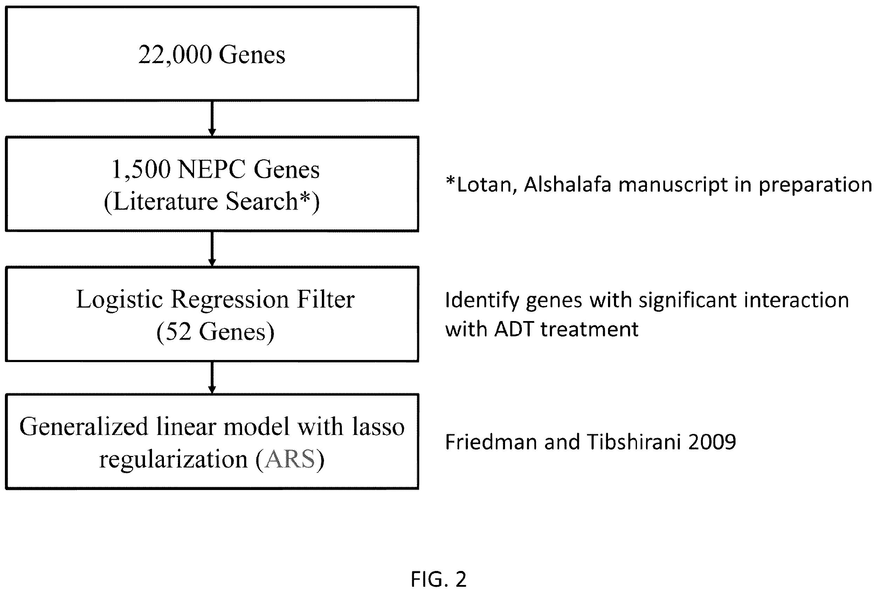

To develop a model to predict subjects' response to ADT (metastasis post treatment), we used 1,557 genes. This list was further filtered using a logistic regression generalized linear model (GLM) function from the stats package using the training cohort. ADT was incorporated as an interaction term with gene expression while adjusting for competing risk. In the process of fitting the GLM different weights were assigned to subject outcomes. Subjects who received salvage hormonal therapy got lower weights (weight < 1), which is inversely correlated with the time they received salvage hormonal therapy, compared to subjects who did not receive salvage hormonal therapy (weights = 1). To identify robust features, we selected genes that were significant (p < 0.05) and resulted in the least model deviance. This filtering process resulted in 52 features for model training (FIG. 2, Table 1).

**Table1: The 52 Genes in the ARS model with their coefficients and p-values**

| **Gene** | **GLM p-values** | **GLM coefficient of treated arm** | **GLM coefficient of untreated arm** |
|---|---|---|---|
| DAND5 | 0.038 | 1.371 | 0 |
| GABRB3 | 0.029 | -3.025 | 1.071 |
| KLK12 | 0.036 | 0 | 0 |
| NAV2 | 0.038 | 0 | 0 |
| POGK | 0.039 | 0 | 0 |
| RIMS2 | 0.021 | -2.854 | 0.849 |
| SNCAIP | 0.028 | 0 | -2.398 |
| TET1 | 0.048 | 0 | 0 |
| TMEM176A | 0.036 | 0.012 | 0 |
| ELL2 | 0.043 | 0 | 0 |
| ADAMTS14 | 0.031 | 0 | 0 |
| CDKN2C | 0.048 | 0 | 0 |
| KCNMB2 | 0.028 | 0.912 | 1.15 |
| MUC1 | 0.017 | 0 | 0 |
| PLEKHH2 | 0.031 | 3.899 | -0.298 |
| TNIK | 0.043 | 0 | 0 |
| AGTR1 | 0.044 | -0.488 | 0.455 |
| BAG3 | 0.033 | 0 | -0.506 |
| C10orf81 | 0.01 | 0.255 | 0 |
| C8orf4 | 0.033 | -0.063 | 0 |
| CRISP2 | 0.032 | 0 | -2.481 |
| GALM | 0.012 | -3.551 | 2.672 |
| GHR | 0.03 | -0.59 | 0.81 |
| POLD4 | 0.016 | 0 | 0 |
| RRAS | 0.033 | 2.19 | 0 |
| SPATA13 | 0.04 | 0 | -3.317 |
| TMEFF2 | 0.044 | 0 | 0.031 |
| ARHGAP11A | 0.036 | 1.323 | 0 |
| ASB16 | 0.043 | 0 | 0 |
| CASP2 | 0.013 | 0 | 0 |
| FAM57B | 0.027 | 0 | 0 |
| FOXM1 | 0.036 | 0 | 0 |
| GNAZ | 0.016 | 0 | -0.432 |
| AC084018.1 | 0.024 | 0 | -0.386 |
| NAV1 | 0.032 | 0 | 0 |
| RRM2 | 0.049 | 0.471 | 0.151 |
| TFAP4 | 0.019 | 2.524 | 0 |
| HEPN1 | 0.026 | 1.581 | 0 |
| KIF1C | 0.036 | 0 | 0 |
| MPDU1 | 0.03 | 0 | -0.1 |
| RLN1 | 0.049 | -1.741 | 0.796 |
| SELE | 0.022 | -5.114 | -0.866 |
| WDR93 | 0.036 | 0 | -0.678 |
| ATF5 | 0.029 | 0 | -0.634 |
| HEY2 | 0.035 | 0.6 | 0 |
| NUP210 | 0.046 | 0 | 0 |
| CDH3 | 0.038 | 0 | 0 |
| CREB3L1 | 0.039 | 0 | -0.339 |
| SLC35F2 | 0.028 | 3.502 | 0 |
| TRPV6 | 0.02 | 0 | 0 |
| FAM134A | 0.02 | 0.259 | 0 |
| NSMCE4A | 0.039 | 0 | -1.025 |

We used a generalized linear model with lasso regularization (GLMLasso (R package glmnet 2.0-2)) optimized using 10-fold cross validation. This resulted in an androgen resistance signature (ARS) model. The ARS generates scores from 0 to 1. Subjects treated with ADT that developed metastasis have higher ARS scores compared to non-metastatic subjects or subjects that did not get ADT.

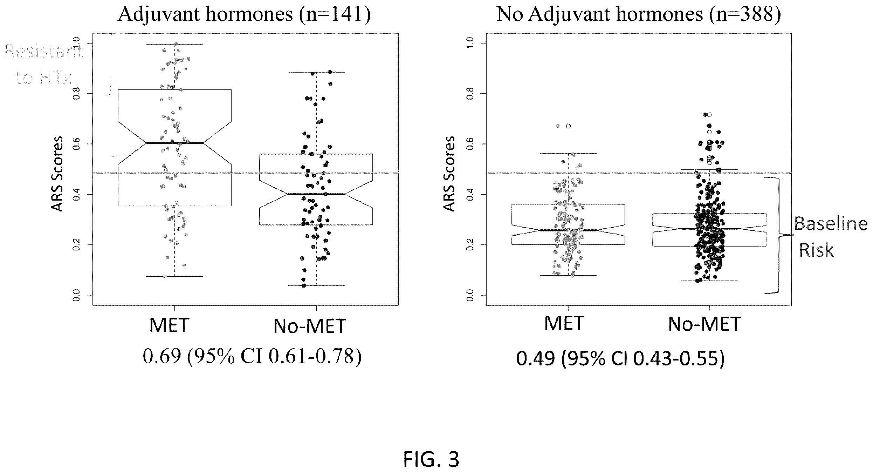

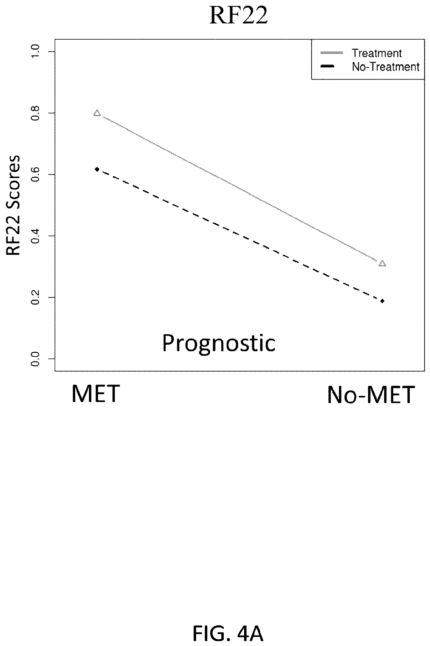

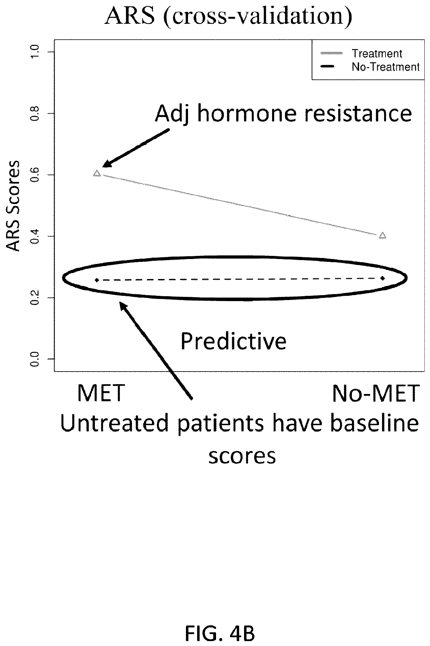

In the cross validation performed in the training cohort, ARS had an area under curve (AUC) of 0.69 (95% CI 0.61-0.78) for predicting metastasis in the adjuvant ADT treated subjects compared to 0.49 (95% CI 0.43-0.55) in the untreated subjects. Subjects treated with ADT who developed metastasis had higher ARS scores based on their primary tumor as compared to subjects who did not have metastasis or subjects who did not get ADT (see FIG. 3). Interaction plots further emphasize that ARS is predictive of ADT failure since ADT resistant subjects had higher scores whereas untreated subjects had baseline scores (see FIG. 4). The interaction plot of Decipher®, a prognostic test developed to predict metastasis post-RP is not significant suggesting that ADT is a predictive, not a prognostic model.

In multivariable analysis, the ARS had a significant interaction with ADT, which showed that the ARS can significantly predict adjuvant hormone treatment failure (see Table 2). These results showed that the methods of the present invention are useful for predicting resistance to androgen deprivation therapy (ADT) in subjects with prostate cancer.

**Table 2: MVA of ARS and clinical variables in cross validation in training cohort**

| **Variables** | **OR (95% CI)** | **P-Value** |
|---|---|---|
| (Intercept) | 0.42 (0.20 - 0.87) | 0.0201 |
| ARS | 0.41 (0.06 - 2.74) | 0.3586 |
| Adj. Hormone | 0.17 (0.05 - 0.53) | 0.0020 |
| Surgical Margins | 0.84 (0.54 - 1.29) | 0.4229 |
| Extraprostatic Extension | 1.42 (0.93 - 2.17) | 0.1016 |
| Seminal Vesicle Invasion | 1.67 (1.04 - 2.66) | 0.0322 |
| Lymph Node Invasion | 2.04 (1.00 - 4.24) | 0.0512 |
| Pathologic GS > 7 (Ref: GS ≤ 7) | 3.85 (2.54 - 5.87) | 1.0e-12 |
| Log2(Preoperative PSA) | 0.90 (0.76 - 1.07) | 0.2413 |
| Adj Radiation 12 months | 2.31 (1.23 - 4.41) | 0.0087 |
| **ARS : (Adj. Hormone)** | **34.07 (2.84 - 445.03)** | **0.0052** |

### Example 2: Systems biology of ARS genes.

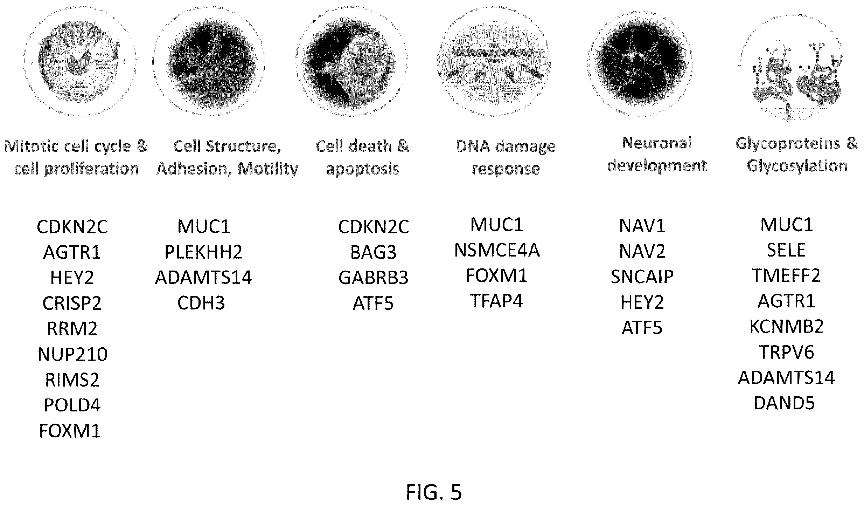

Functional characterization of the genes in the ARS model showed that the genes are involved in multiple biological pathways and biological processes involved in cancer progression and neuronal development (FIG. 5). Several genes such as NSMCE4A, FOXM1, TFAP, GABRB3, and ATF5 are associated with DNA damage response, and apoptosis signaling pathway. Furthermore, some of the genes have evidence of being involved in androgen signaling. For example, HEY2 is reported to be able to specifically repress AR-dependent transcriptional activity (Villaronga et al. (2008) Curr. Cancer Drug Targets 8(7):566-580). Other ARS genes including RRM2 and CRISP2 play roles in cell cycle and cell proliferation. RRM2 has a role in proliferation, invasion of prostate cancer, and when over-expressed, is associated with resistance to gemcitabine and platinum-based chemotherapy in other cancers (Wang et al. (2014) Tumour Biol. 35(3):1899-1906). E-selectin (SELE) has an important role in cell adhesion and controls circulating prostate cancer cell adhesion and bone metastasis (Yasmin-Karim et al. (2014) Oncotarget 5(23):12097-12110).

### Example 3: Independent Validation of ARS to Predict Hormone Treatment Failure after Adjuvant Hormone Treatment.

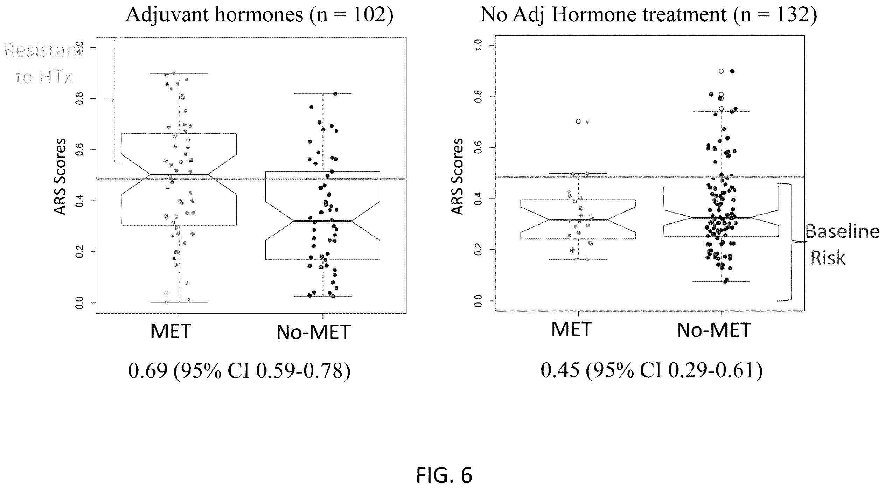

The ARS model was validated on an independent cohort from Mayo Clinic II (n=233) (Karnes et al (2013) J. Urol. 190(6):2047-2053). This cohort is a case-cohort design with 101 treated subjects, 51 subjects who metastasized, and 132 subjects who did not receive ADT, which had 24 metastatic subjects (Karnes et al., *supra*). The same tissue selection and sample processing was applied as in Example 1. In this independent validation, the ARS had a 10-year survival c-index of 0.69 (95% CI 0.59-0.78) in the adjuvant ADT treated subjects compared to 0.45 (95% CI 0.29-0.61) in the untreated subjects (FIG. 6). Treated subjects who metastasized had high scores compared to untreated subjects who had base line scores. These results demonstrated that treated subjects with higher ARS scores tend to fail treatment and would be good candidates for other treatment modalities. These results further showed that the methods of the present invention are useful for predicting resistance to androgen deprivation therapy (ADT) in subjects with prostate cancer.

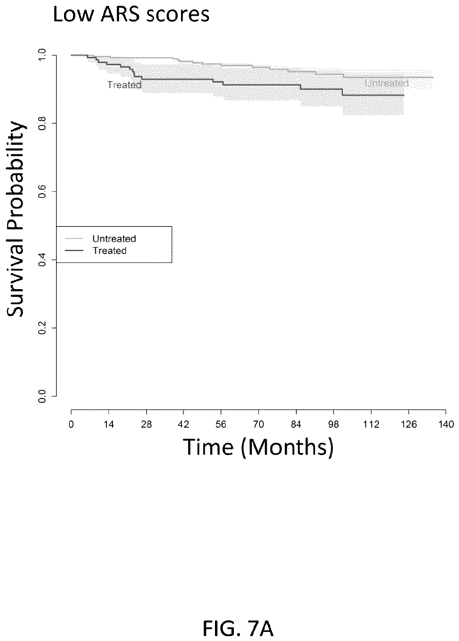

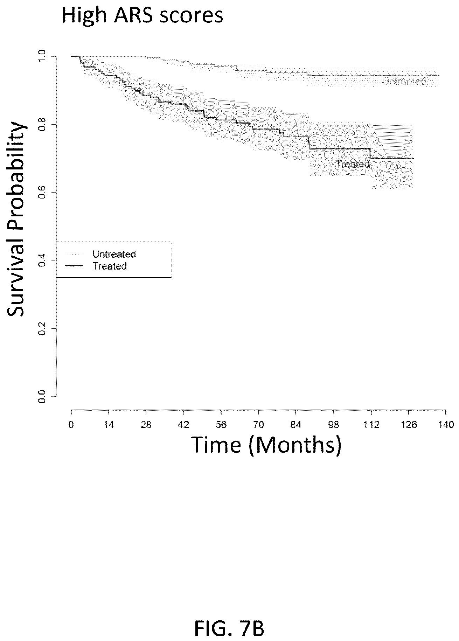

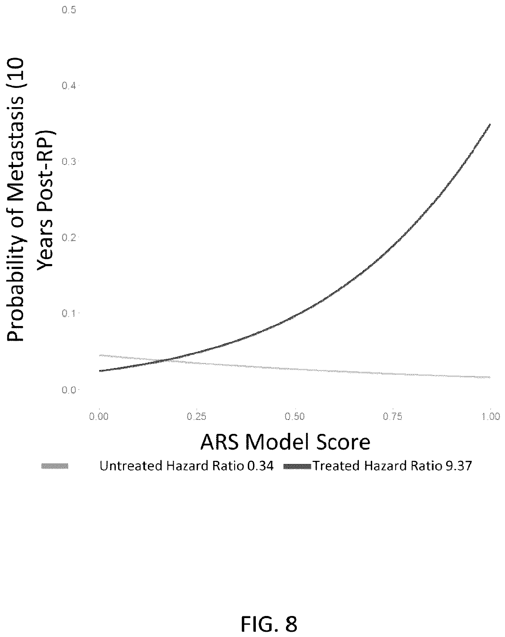

In another series of experiments, the survival outcomes of metastatic (Met) and non-metastatic (No-Met) subjects in Kaplan-Meier analysis of low and high ARS score groups in the Mayo Clinic II cohort was compared (FIG. 7). In the high ARS score group, Kaplan-Meier analysis indicated 10-year metastasis-free survival of 70% and 95% (Bonferroni log-rank, p < 0.001), whereas in the low ARS group they were 87% and 93% comparing treated and untreated subjects, respectively (p=0.086). In multivariable analysis ARS had a significant interaction with adjuvant treatment (p=0.022) (Table 3) and the hazard ratio among treated subjects was 9.37 compared to 0.34 among untreated subjects for every 10% increase in ARS score (FIG. 8). These results showed that the methods and classifiers of the present invention are useful for predicting resistance to androgen deprivation therapy (ADT) in subjects with prostate cancer.

**Table 3: MVA of ARS and clinical variables in independent validation cohort**

| **Variables** | **Hazard Ratio** | **P-Value** |
|---|---|---|
| ARS | 0.34 | 0.464 |
| Adj. Hormone | 0.52 | 0.377 |
| Surgical Margins | 0.98 | 0.957 |
| Extraprostatic Extension | 1.52 | 0.234 |
| Seminal Vesicle Invasion | 1.64 | 0.165 |
| Lymph Node Invasion | 0.74 | 0.513 |
| Pathologic GS > 7 (Ref: GS ≤ 7) | 2.08 | 0.024 |
| Log2(Preoperative PSA) | 1.14 | 0.324 |
| Adj Radiation | 1.65 | 0.193 |
| ARS : (Adj. Hormones) | 53.25 | 0.022 |

### Example 4: Evaluating ARS in a Natural History Cohort with No Onset of Treatment.

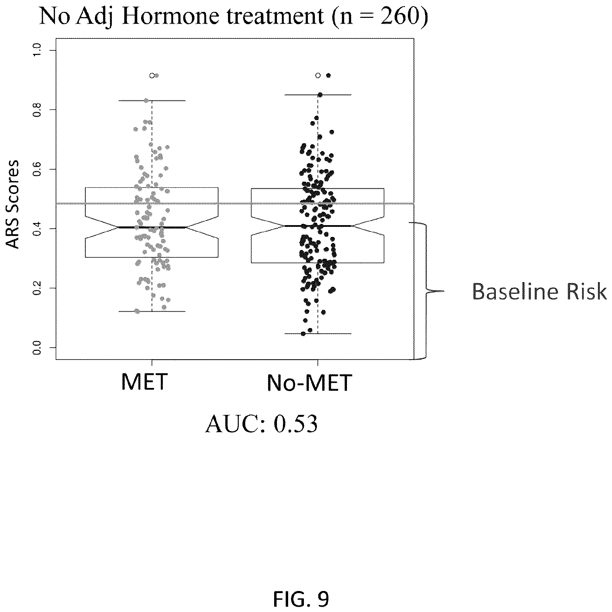

The ARS model was evaluated in a case-cohort of 260 subjects with no ADT treatment until the time of metastasis from John Hopkins Medical Institute (n=260) (Ross et al. (2015) Eur. Urol. Jun 6, pii:S0302-2838(15)00444-3). 99 out of 260 subjects developed metastasis. Tissue selection and sample processing were applied as described in Example 1. ARS was not prognostic of metastasis in this cohort (c-index 0.53) (FIG. 9). The distribution of ARS scores was not different between subjects with metastatic outcome and subjects with no metastasis, and they were similar to baseline scores. These results showed that ARS predicts metastasis after ADT treatment.

### Example 5: ARS is Associated with Rapid Metastatic Castration Resistance.

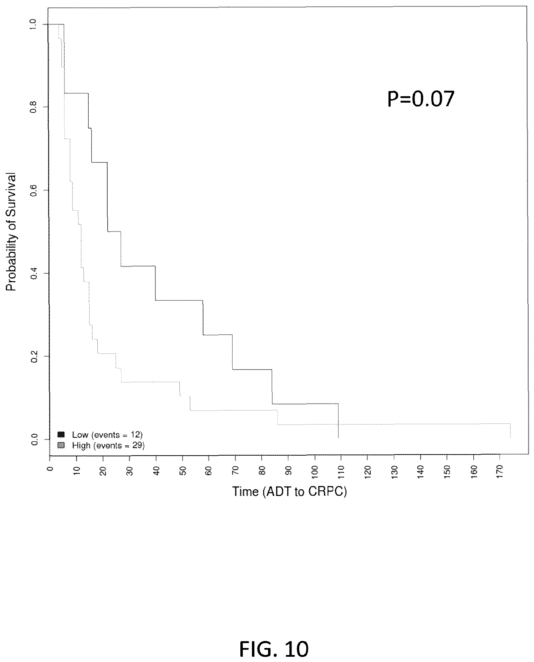

The association of ARS with rapid metastatic castration resistance was examined as follows. Out of the 99 subjects who developed metastasis in the JHMI cohort (n=260) described above in Example 4, 52 developed metastatic castration resistant prostate cancer (mCRPC) after receiving post metastatic hormonal therapy. Subjects with higher ARS scores were associated with rapid metastatic castration resistance (p=0.07) with median ADT to m-CRPC time of 13 months compared to 28 months for subjects with low ARS (FIG. 10). These results showed that the methods and classifiers of the present invention are useful for diagnosing metastatic castration resistant prostate cancer (mCRPC) in subjects with prostate cancer.

### Example 6: Evaluating ARS in Small Cell and Neuroendocrine Prostate Cancer.

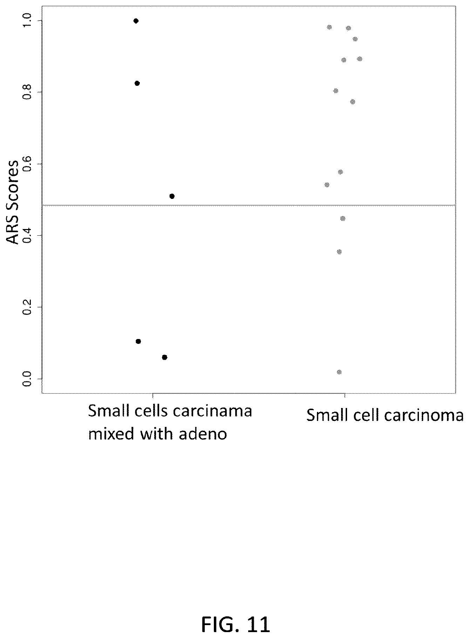

The ARS model was evaluated in 17 small cell (SC) prostate cancer samples from JHMI. These samples were not included in the ARS development. ARS scores of the SC samples were mostly very high; 12 out of 17 had scores above 0.5 suggesting that these samples will fail hormonal treatment if received (FIG. 11). These results suggest that SC samples lack androgen signaling, and ADT, therefore, is not effective for them.

### Example 7: Evaluating ARS in Biopsy Setting.

Although the ARS model was developed with radical prostatectomy (RP) tissue, the ARS is applicable to biopsy tissues generally and should provide an indication of whether or not a tumor will respond to first line hormonal therapy (e.g., bicalutamide). Initial analysis showed that ARS scores are correlated between matched biopsy and RP tissues suggesting that the ARS is of clinical benefit in a biopsy setting.

### Example 8: ARS Improves Treatment Decision Making.

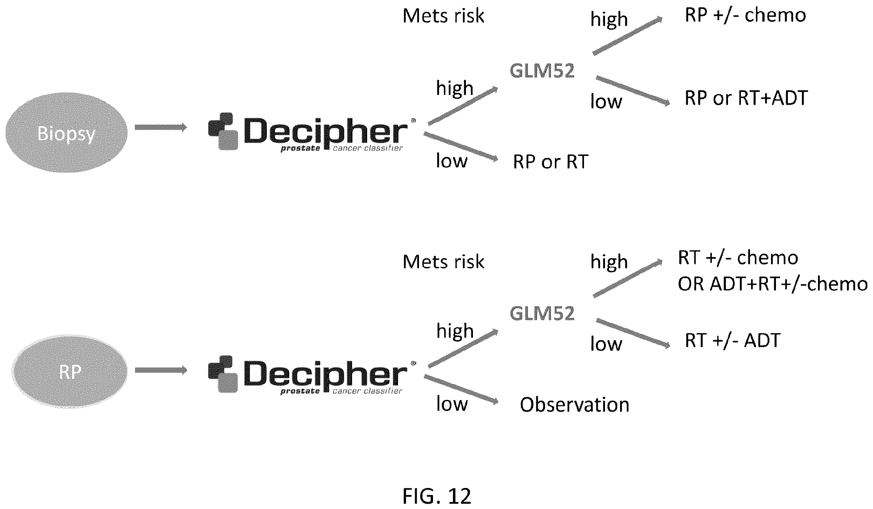

The ARS model is used in a genomic paradigm for better treatment management. The ARS is applied to subjects with high Decipher scores who are at higher risk of developing metastasis and require secondary treatment. Subjects with high ARS and high Decipher scores will most probably fail ADT, and a combination of ADT and radiation therapy, and/or chemotherapy, and/or radiation therapy, and/or chemotherapy may be a better treatment option. Subjects with high Decipher® and low ARS scores should respond better to a combination of ADT and radiation therapy because subjects who have high Decipher® scores have been shown to respond better to radiation therapy (FIG. 12).

### Example 9: Development and Assessment of a Genomic Classifier to Predict Hormone Treatment Failure.

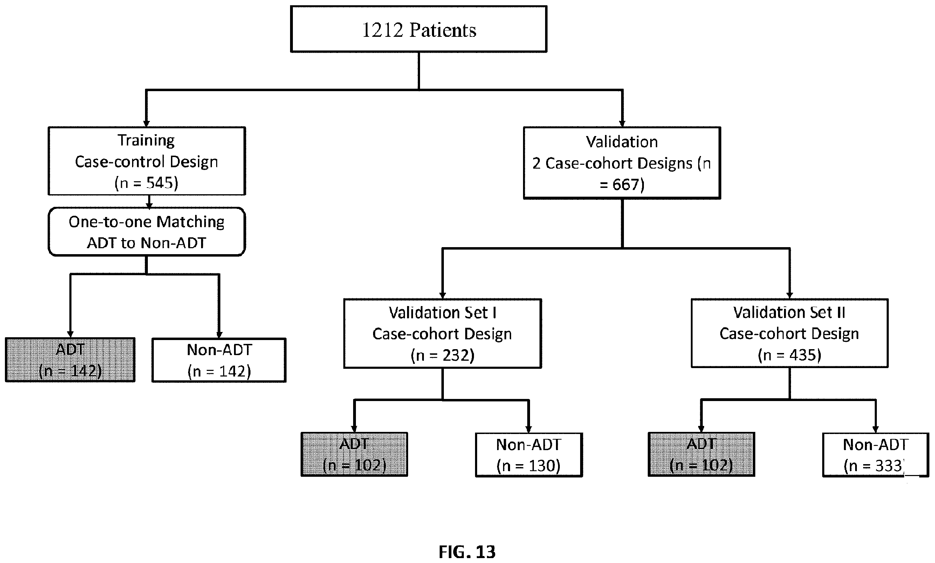

A genomic classifier for predicting hormone treatment failure was developed and assessed as follows. A total of 284 patients PCa expression profiles from FFPE tissues were analyzed from the Decipher GRID database and was used as a training set to develop an Androgen Deprivation Therapy (ADT) Resistance Signature (ARS). Training data was collected from a nested case-control design cohort from a tumor registry. Patients with hormonal treatment within 12 months of radical prostatectomy (RP) defined the adjuvant ADT arm and patients with treatment after 12 months or no treatment defined the no treatment arm. ADT-treated patients were matched using propensity scores to non-ADT treated patients. As a result, 142 patients were in the ADT treated arm, and 142 patients were in the no treated arm (Figure 13).

A representative FFPE block from the index prostate cancer lesion was identified by pathologists. RNA was extracted from tumor tissues from the representative FFPE blocks, amplified using the Ovation WTA FFPE system (NuGen, San Carlos, CA, USA), labeled and hybridized to Human Exon 1.0 ST microarrays (Affymetrix, Santa Clara, CA) covering 1.4 million probesets that were summarized to ∼46,000 gene expression profiles. Analysis and generation of signature scores were performed as previously described in (Erho et al. 2013) and Quality control was verified using Affymetrix power tools (Lockstone 2011). All data normalization used the SCAN algorithm (Piccolo et al. 2014).

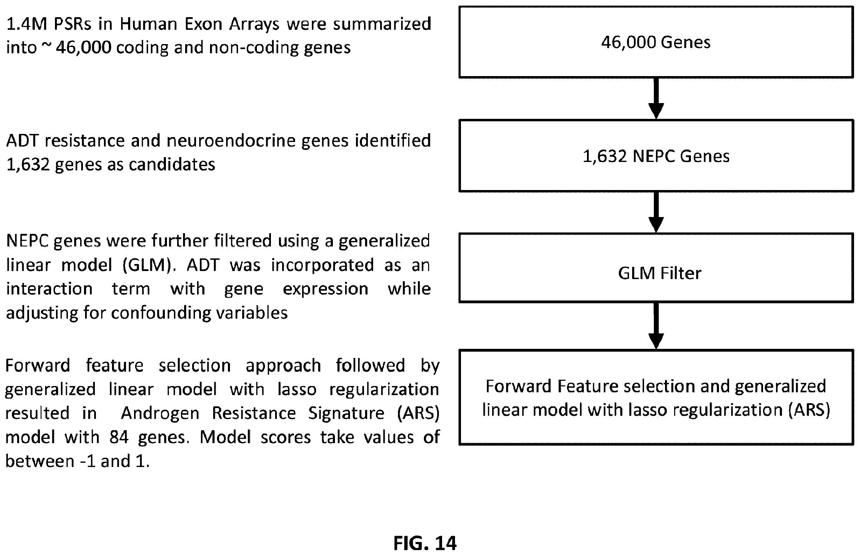

To develop a model to predict patients' response to ADT (metastasis post treatment), we used the 1,632 genes described in Example 1. This list was further filtered using a logistic regression generalized linear model (GLM) function from the stats package using the training cohort. ADT was incorporated as an interaction term with gene expression while adjusting for competing risk. Then, Forward feature selection approach using generalized linear model with lasso regularization resulted in an Androgen Resistance Signature (ARS) model with 84 genes. Model scores take values between - 1 and 1 (See Figure 14 and Table 4).

**Table 4: The 84 Genes in the ARS model with their coefficients**

| Gene | GLM coefficient of treated arm | GLM coefficient of no treated arm |
|---|---|---|
| SELE | -6.71642 | 0.81019 |
| FAM134A | 3.341691 | 0.503562 |
| FBXO43 | -3.12012 | 2.518364 |
| SLC35F2 | 6.247134 | 0 |
| CLEC9A | 4.843049 | -1.59313 |
| CRYM | -6.14733 | 0 |
| GABRB3 | -1.69443 | 5.360194 |
| RBPMS2 | 2.278909 | 0 |
| HEPN1 | 0.583004 | -0.08359 |
| HEY2 | 1.357111 | 0 |
| C8orf4 | -0.79372 | -0.03234 |
| ALDH2 | 0 | -0.49546 |
| MPP7 | 0 | 0 |
| CFL1 | 0 | 0 |
| DESI2 | 0 | 0 |
| OR51E2 | 0 | 0 |
| LRRC18 | 1.5042 | -2.42353 |
| KCNMB1 | 0 | 0 |
| DLGAP1 | 0 | 0 |
| SPRR1A | 0 | 0 |
| CROT | 0 | 0 |
| KIFC1 | 0 | 0 |
| POLD4 | 0 | 0 |
| MSMB | -0.1096 | 0 |
| PEX11A | 3.915103 | 0 |
| CASP2 | 0 | 0 |
| GALM | -3.03597 | 1.7822 |
| WHSC1 | 0 | 0 |
| MPZL2 | 0 | 0 |
| NAV1 | 0 | 0 |
| KLK12 | 0 | -0.0653 |
| PRTFDC1 | 0 | -0.75669 |
| HJURP | 1.597005 | 0 |
| RNF168 | 0 | 0 |
| CDH26 | -1.8008 | 0.798799 |
| FOXM1 | 0 | 0 |
| ZC3H11A | 0 | 0 |
| NPR3 | -1.49893 | 0 |
| PRKAG1 | 0 | -6.33891 |
| FAM3D | 0 | 0 |
| KCNK17 | 0 | 0 |
| PLXNA2 | 0 | 0 |
| SUOX | 0 | 0 |
| KIAA1210 | 0 | -3.9371 |
| ANP32E | 0 | 0 |
| TMEM133 | 1.221927 | -3.45756 |
| TM2D2 | 0 | 2.777631 |
| REST | 0 | 0 |
| LPGAT1 | 2.855496 | 0 |
| FAHD2A | -0.37686 | 0 |
| NKX2-2 | 0 | 0 |
| NAV2 | 2.178303 | 4.208435 |
| CCDC151 | 0.420145 | -3.35072 |
| KCNH8 | -4.02515 | 0 |
| TXK | 0 | -2.78639 |
| RBBP8 | 0 | 0 |
| RLN1 | -2.36752 | 0.436944 |
| NSMCE4A | 0 | 0 |
| DNAJC12 | -0.07306 | 2.720789 |
| H19 | 0 | 0 |
| SOX14 | -1.29956 | 0.234591 |
| ATP1A2 | 0 | 0 |
| TACC2 | 0 | -0.19549 |
| PLXNC1 | 0 | 0 |
| NUP62 | 0 | 0 |
| SFTA3 | 0.836221 | 0 |
| EHHADH | 0 | -2.95172 |
| LSM7 | 0 | 0.382088 |
| B3GALTL | -7.03208 | 0.301311 |
| ID2 | 0 | 0.592488 |
| ACAA2 | 0 | 0 |
| ADH1C | 0 | 0 |
| STMN1 | 1.937662 | 4.039599 |
| TFAP4 | 4.531512 | 0 |
| SEMA3C | 0.492556 | -1.33672 |
| THYN1 | 0 | 0 |
| GLYATL1 | -3.46113 | 0 |
| NUDT1 | 0 | -2.21441 |
| CCL16 | 0 | -1.82467 |
| EZH2 | 0 | -2.03534 |
| NLRP13 | 0 | -0.02317 |
| COX7A2L | 0 | 0 |
| MAP1B | 0 | 0 |
| MORN3 | 0 | -3.7122 |

### Example 10: Independent Validation of ARS to Predict Hormone Treatment Failure After Adjuvant Hormone Treatment.

The ARS model in Example 9 was validated using an independent cohort (n=232) (validation set I) with 102 treated patients, and 130 patients who did not receive ADT [Karnes et al. 2013]. The tissue selection and sample processing was the same as in Example 2 above. In multivariable analysis, the ARS had a significant interaction with ADT. These results show that the ARS can significantly predict adjuvant hormone treatment failure (Table 5). These results further showed that the methods and classifiers of the present invention are useful for predicting resistance to androgen deprivation therapy (ADT) in subjects with prostate cancer.

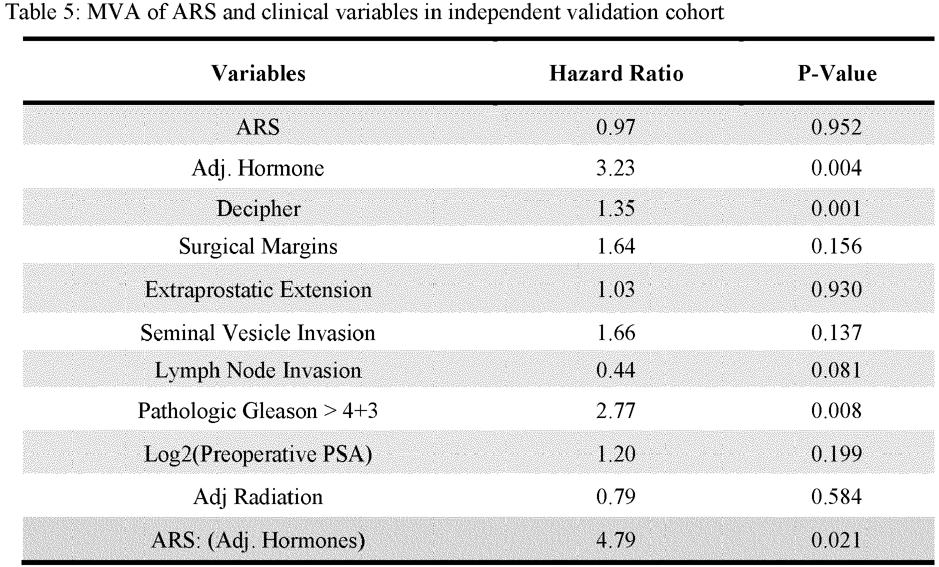

### Example 11: Evaluating ARS in a Cohort Enriched with Patients from a Treatment-Naïve Case-Cohort Study.

The ARS model in Example 9 was evaluated in a cohort enriched with patients from a treatment- naïve case-cohort as follows. Validation set I was enriched with a cohort enriched with patients from a treatment-naive case-cohort study (hereinafter, validation set II). The treatment-naive case-cohort consisted of 260 patients with no ADT treatment till the time of metastasis (n=260) (Ross et al, 2015). Ninety-nine out of 260 patients developed metastasis. Tissue selection and sample processing was the same as in Example 2 above. Validation Set II had 102 treated patients and 333 patients who did not receive ADT.

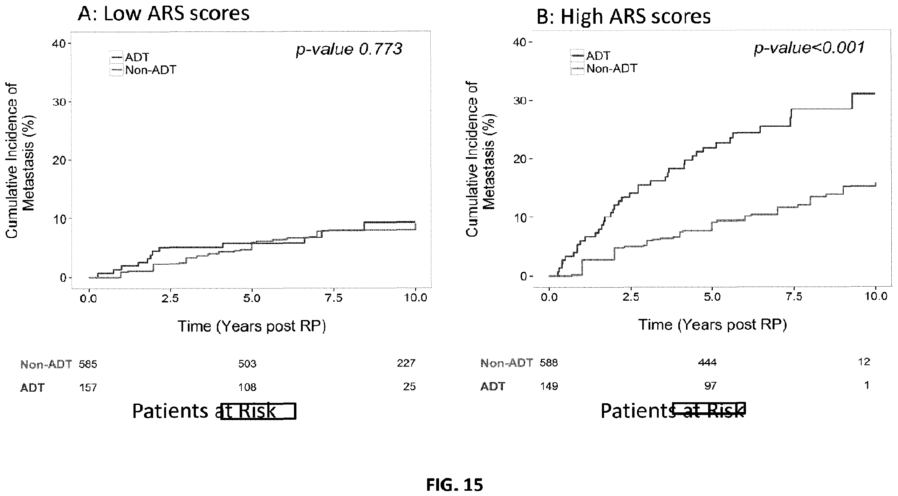

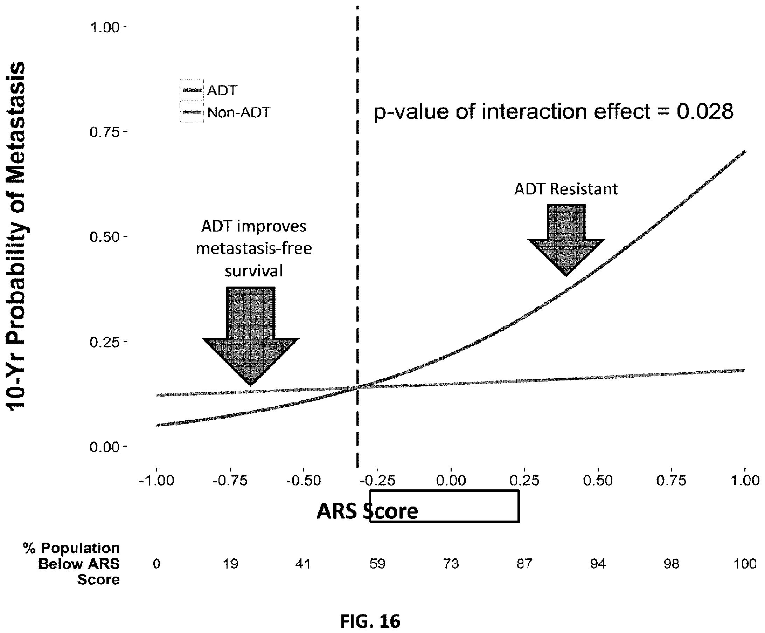

We next compared the survival outcomes of metastatic (Met) and non-metastatic (No-Met) patients (see Figure 15). In the high ARS score group, patients receiving ADT had significantly higher incidence of metastasis (p < 0.001), whereas in the low ARS group incidence of metastasis was the same between treated and untreated patients (p=0.773). In multivariable analysis ARS had a significant interaction with ADT (p=0.028) (Table 6). Moreover, Risk-adjusted 10-year prediction curves showed that treated patients with higher ARS scores have higher probability of developing metastasis (p-value of interaction effect = 0.028) (Figure 16). These results further showed that the methods and classifiers of the present invention are useful for predicting resistance to androgen deprivation therapy (ADT) in subjects with prostate cancer.

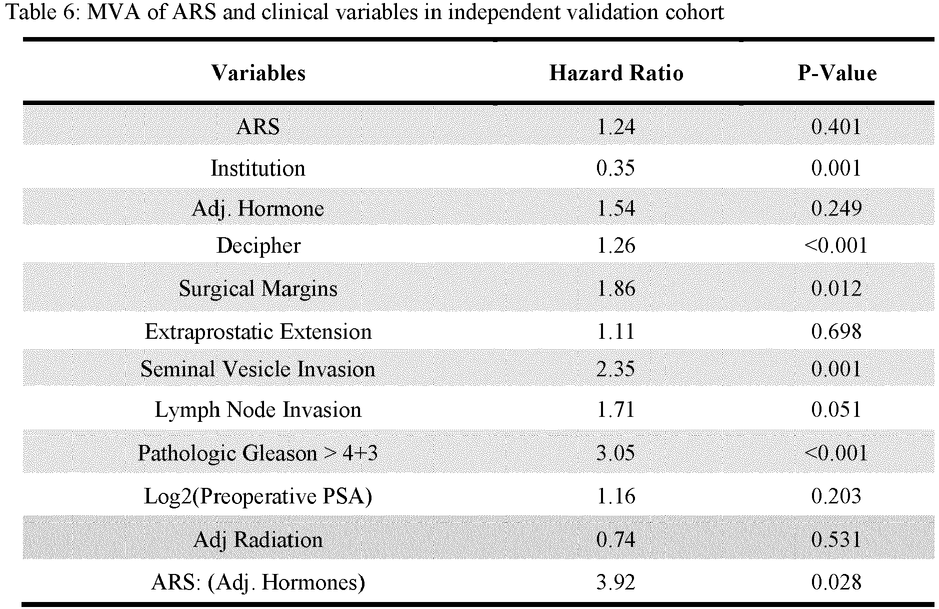

## Claims

1. A diagnostic method for predicting resistance to androgen deprivation therapy (ADT) for a subject who has prostate cancer, the method comprising:
(a) measuring the level of expression of a plurality of biomarker genes comprising SELE, PRKAG1, B3GALTL, CRYM, NAV2, GABRB3, CLEC9A, SLC35F2, GALM, FBXO43, TMEM133, ID2, KCNH8, EZH2, RLN1, CDH26, SOX14, MSMB, TXK, STMN1, OR51E2, DESI2, TFAP4, KIAA1210, LRRC18, PEX11A, CCDC151, MORN3, GLYATL1, EHHADH, LPGAT1, FAM134A, DNAJC12, TM2D2, RBPMS2, NUDT1, SEMA3C, CCL16, HJURP, NPR3, HEY2, SFTA3, C8orf4, PRTFDC1, HEPN1, ALDH2, LSM7, FAHD2A, TACC2, KLK12, NLRP13, MPP7, CFL1, KCNMB1, DLGAP1, SPRR1A, CROT, KIFC1, POLD4, CASP2, WHSC1, MPZL2, NAV1, RNF168, FOXM1, ZC3H11A, FAM3D, KCNK17, PLXNA2, SUOX, ANP32E, REST, NKX2.2, RBBP8, NSMCE4A, H19, ATP1A2, PLXNC1, NUP62, ACAA2, ADH1C, THYN1, COX7A2L and MAP1B in a biological sample obtained from a subject; and
(b) calculating an ADT resistance signature (ARS) score based on the level of expression of the plurality of biomarkers, wherein a higher ARS score for the subject compared to reference value ranges for a control subject indicates that the subject is resistant to ADT.

2. The method of claim 1, wherein the subject is undergoing ADT.

3. The method of claim 1, wherein the method is performed after treatment of the subject with ADT or after the subject undergoes radical prostatectomy.

4. The method of claim 1, wherein the prostate cancer is adenocarcinoma, small cell prostate cancer, neuroendocrine prostate cancer or metastatic castration resistant prostate cancer

5. The method of claim 1, wherein the biological sample is a biopsy or a tumor sample.

6. The method of claim 1, wherein measuring the level of expression comprises performing microarray analysis, polymerase chain reaction (PCR), reverse transcriptase polymerase chain reaction (RT-PCR), a Northern blot, or serial analysis of gene expression (SAGE).

## Patentansprüche

1. Diagnostisches Verfahren zur Vorhersage der Resistenz gegenüber Androgendeprivationstherapie (ADT) für ein Individuum, das an Prostatakrebs leidet, wobei das Verfahren Folgendes umfasst:
(a) Messen des Expressionsniveaus von mehreren Biomarker-Genen, die SELE, PRKAG1, B3GALTL, CRYM, NAV2, GABRB3, CLEC9A, SLC35F2, GALM, FBX043, TMEM133, ID2, KCNH8, EZH2, RLN1, CDH26, SOX14, MSMB, TXK, STMNl, OR51E2, DESI2, TFAP4, KIAA1210, LRRC18, PFXllA, CCDC151, MORN3, GLYATLl, EHHADH, LPGATl, FAM134A, DNAJC12, TM2D2, RBPMS2, NUDTl, SEMA3C, CCL16, HJURP, NPR3, HEY2, SFTA3, C8orf4, PRTFDC1, HEPN1, ALDH2, LSM7, FAHD2A, TACC2, KLK12, NLRP13, MPP7, CFL1, KCNMBl, DLGAP1, SPRR1A, CROT, KIFC1, POLD4, CASP2, WHSC1, MPZL2, NAV1, RNF168, FOXM1, ZC3H11A, FAM3D, KCNK17, PLXNA2, SUOX, ANP32E, REST, NKX2.2, RBBP8, NSMCE4A, H19, ATP1A2, PLXNC1, NUP62, ACAA2, ADH1C, THYN1, COX7A2L und MAP1B umfassen, in einer aus einem Individuum erhaltenen biologischen Probe und
(b) Berechnen eines Scores für die ADT-Resistenz-Signatur (ARS) auf der Basis des Expressionsniveaus der mehreren Biomarker, wobei ein höherer ARS-Score für das Individuum im Vergleich zu Referenzwertbereichen für ein Kontrollindividuum anzeigt, dass das Individuum gegenüber ADT resistent ist.

2. Verfahren nach Anspruch 1, wobei das Individuum ADT durchläuft.

3. Verfahren nach Anspruch 1, wobei das Verfahren nach Behandlung des Individuums mit ADT oder nach radikaler Prostatektomie des Patienten durchgeführt wird.

4. Verfahren nach Anspruch 1, wobei es sich bei dem Prostatakrebs um Adenokarzinom, kleinzelligen Prostatakrebs, neuroendokrinen Prostatakrebs oder metastatischen kastrationsresistenten Prostatakrebs handelt.

5. Verfahren nach Anspruch 1, wobei es sich bei der biologischen Probe um eine Biopsie oder eine Tumorprobe handelt.

6. Verfahren nach Anspruch 1, wobei das Messen des Expressionsniveaus das Durchführen von Mikroarray-Analyse, Polymerasekettenreaktion (PCR), Reverse-Transkriptase-Polymerasekettenreaktion (RT-PCR), einem Northern Blot oder SAGE (Serial Analysis of Gene Expression) umfasst.

## Revendications

1. Procédé diagnostique pour prédire la résistance à une thérapie par privation androgénique (ADT) pour un sujet qui a un cancer de la prostate, le procédé comprenant :
(a) la mesure du niveau d'expression d'une pluralité de gènes biomarqueurs comprenant SELE, PRKAG1, B3GALTL, CRYM, NAV2, GABRB3, CLEC9A, SLC35F2, GALM, FBXO43, TMEM133, ID2, KCNH8, EZH2, RLN1, CDH26, SOX14, MSMB, TXK, STMN1, OR51E2, DESI2, TFAP4, KIAA1210, LRRC18, PEX11A, CCDC151, MORN3, GLYATL1, EHHADH, LPGAT1, FAM134A, DNAJC12, TM2D2, RBPMS2, NUDT1, SEMA3C, CCL16, HJURP, NPR3, HEY2, SFTA3, C8orf4, PRTFDC1, HEPN1, ALDH2, LSM7, FAHD2A, TACC2, KLK12, NLRP13, MPP7, CFL1, KCNMB1, DLGAP1, SPRR1A, CROT, KIFC1, POLD4, CASP2, WHSC1, MPZL2, NAV1, RNF168, FOXM1, ZC3H11A, FAM3D, KCNK17, PLXNA2, SUOX, ANP32E, REST, NKX2.2, RBBP8, NSMCE4A, H19, ATP1A2, PLXNC1, NUP62, ACAA2, ADH1C, THYN1, COX7A2L et MAP1B dans un échantillon biologique obtenu d'un sujet ; et
(b) le calcul d'un score de signature de résistance à l'ADT (ARS) sur la base du niveau d'expression de la pluralité de biomarqueurs, un score ARS plus élevé pour le sujet, par comparaison à des plages de valeurs de référence pour un sujet témoin, indiquant que le sujet est résistant à l'ADT.

2. Procédé selon la revendication 1, dans lequel le sujet subit une ADT.

3. Procédé selon la revendication 1, le procédé étant mis en œuvre après traitement du sujet par une ADT ou après que le sujet a subi une prostatectomie radicale.

4. Procédé selon la revendication 1, dans lequel le cancer de la prostate est un adénocarcinome, un cancer de la prostate à petites cellules, un cancer de la prostate neuroendocrinien ou un cancer de la prostate résistant à une castration métastatique.

5. Procédé selon la revendication 1, dans lequel l'échantillon biologique est une biopsie ou un échantillon de tumeur.

6. Procédé selon la revendication 1, dans lequel la mesure du niveau d'expression comprend la mise en œuvre d'une analyse par micro-réseau, d'une réaction en chaîne par polymérase (PCR), d'une réaction en chaîne par polymérase avec transcriptase inverse (RT-PCR), d'un transfert de Northern ou d'une analyse en série de l'expression génique (SAGE).
